# EUROPEAN PATENT APPLICATION

(11) **EP 0 846 761 A1**
(43) Date of publication of application: **10.06.1998**
(21) Application number: 96203482.3
(22) Date of filing: 09.12.1996
(51) Int. Cl.: C12N 15/12, C12N 15/11, C12N 15/86, C12N 5/10, C07K 14/47, C12P 21/02, C12P 21/08, A61K 39/395, A61K 38/17

(54) **A novel transcription factor expressed in cycling cells, nucleic acid molecules encoding said transcription factor and its promoter region and uses thereof**

(71) Applicant: Introgene B.V., NL-2288 GJ Rijswijk (NL)
(72) Inventor: The designation of the inventor has not yet been filed
(74) Representative: Smulders, Theodorus A.H.J., Ir.

(57) **Abstract**

A novel transcription factor and its encoding nucleic acid molecules, as well as its consensus DNA binding motif are disclosed. The invention also discloses the DNA promoter region of the novel transcription factor that drives ubiquitous expression of genes in cycling cells, but is silent in resting cells, and its use in gene therapeutic strategies for the treatment of e.g. malignancies. Furthermore, monoclonal antibodies reacting with said transcription factor and their use for the detection of cycling cells are disclosed.

## Description

The present invention lies in the field of molecular biology and genetic engineering. It particularly relates to proteins involved in the regulation of gene expression and with their nucleic acid counterparts.

During progression of eukaryotic cells through cell cycle large alterations occur in cellular structures and activities. An integrated cascade of phosphorylation-dependent signals regulates the expression of genes requisite for initiation of proliferation and competency for cell-cycle progression. Cyclins are synthesized and activated in a cell cycle-dependent manner and function as regulatory submits of cyclin-dependent kinases, that phosphorylate a broad spectrum of structural proteins and transcription factors, to mediate sequential parameters of cell-cycle control. Passage through the G2 checkpoint is governed by the mitosis-promoting factor (MPF)-complex that is composed of protein kinase p34^{cdc2} and cyclin -B (reviewed by Nurse, Nature 344(1990):503-508). Upon entry into M-phase, many proteins are phosphorylated either directly by MPF or indirectly by kinases that are activated by MPF. Davis et al. (Proc. Natl. Acad. Sci. USA 80(1983):2926-2930) generated the monoclonal antibody (MoAb) MPM2 that binds a phospho amino acid containing epitope on more than fourty different proteins that are phosphorylated during M-phase of the cell cycle. Only very few of these MPM2-reactive proteins have been identified (Vandre et al., J. Cell. Sci. 98(1991):577-588; Tombes et al., Cell Regul. 2(1991):861-874). In a search for putative M-phase phosphoproteins Westendorf et al. (Proc. Natl. Acad. Sci. USA 91(1994):714-718) screened proteins expressed in two cDNA libraries made from RNA of growing HeLa cells and MOLT-4 cells, respectively, after phosphorylation with extracts from M-phase enriched HeLa cells with the MPM2 MoAb. Two partial-length cDNAs were isolated and the putative M-phase phosphoproteins encoded by these cDNAs were named MPP1 and MPP2, respectively.

Among other factors, progression through the cell cycle depends on controlled onset of specific genetic programs. The expression of the genes involved is switched on due to the action of transcription factors that bind to the DNA in the proximity of the transcription start site of these genes, the so-called promoter region. Activation of transcription is caused by a complex interplay between 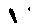 basal*'* transcription factors that bind to the TATA-box of the promoter region and 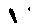 activating*'* transcription factors that bind upstream of the TATA-box. The basal transcription factors bind to the TATA-box in an ordered fashion, forming a multi-subunit transcription initiation complex (TIC), which includes RNA-polymerase II. However, the assembly of the TIC occurs at a slow rate. Most activating transcription factors contain a DNA-binding domain, with which they bind to specific DNA sequences. In addition, they contain transactivating domains, with which they bind to certain members of the TIC. When bound to specific DNA sequences in the proximity of the TATA-box, the activating transcription factors accelerate rate limiting steps in the assembly of the TIC. They recruit basal transcription factors, e.g. basal 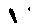 transcription factor II B' by physically interacting with them. This speeds up the assembly of the TIC. Moreover, certain activating transcription factors induce DNA bending, which also contributes to enhanced initiation of transcription. Based on the structure of their DNA-binding domains, the activating transcription factors can be classified into several families: 1) Helix-loop-helix proteins (e.g., CTF1/NF1), 2) Helix-turn-helix proteins (e.g., Oct1), 3) Zinc finger proteins (e.g., Sp1), 4) bZip proteins (e.g., c-Jun, Fos), 5) Rel-related proteins (e.g., NFkB), and 6) Winged-helix or fork head proteins.

The family of fork head (fkh) proteins is a large family of transcription factors, characterized by a highly conserved DNA-binding domain with various biological functions. The prototype of this group is the product of the homeotic *Drosophila melanogaster* gene *fork head* (Weigel et al., Cell 57(1989):645-648), which is required for the terminal development of the Drosophila embryo. Comparison of the sequences of *fork head* and the Hepatocyte Nuclear Factor-3a (HNF-3a), a protein involved in liver-specific gene expression in the rat (Lai et al., Genes Dev. 4(1990):1427-1436) revealed a novel, highly conserved DNA-binding protein motif of 110 amino acids. The DNA-binding specificity of several members of the fkh family has been determined. For four human and three rat fkh proteins single core sequences of six or seven nucleotides were found (Pierrou, et al, EMBO J. 13(1994):5002-5012; Overdier, et al, Mol. Cell. Biol. 14(1994):2755-2766). Subtle differences at the 3' and 5' termini of the consensus sites allow distinct binding and transcriptional activation of different family members. The promoter of the lung-specific gene *CC10*, for example, allows various fkh family members to bind *in vitro* and *in vivo*. However, from the tested proteins only FREAC-1 appeared to be a strong activator of the *CC10* gene in a lung cell line (Hellqvist, et al., J. Biol. Chem. 271(1996):4482-4490).

After the elucidation of the butterfly-like co-crystal structure of HNF-3g bound to DNA (Clark et al., Nature 364(1993):412-420) the members of the fkh family are also referred to as "winged-helix" proteins. According to the organization of a-helices and b-turns the winged-helix motif can be classified as a variant of the helix-turn-helix motif (reviewed by Brennan, Cell 74(1993):773-776). Vuister et al. (Biochemistry 33(1994):10-16) reported NMR evidence for similarities between the DNA-binding domains of HNF-3g and the Drosophila heat shock factor. The secondary structure of the heat shock factor and the pattern of residues involved in DNA binding are similar to those observed for the complexes between DNA and HNF-3g (Clark et al., Nature 364(1993):412-420). Although HNF-3g, like all known members of the fkh family, binds DNA as a monomer, the structure of the HNF-3g-DNA complex was elucidated as an asymmetric unit containing two oligonucleotides in a 5'-3' ,3'-5' orientation and two HNF-3g molecules bound in a tail-to-tail order. This resembles the binding of heat shock factors to DNA heat shock elements, which often occurs in multimers. These heat shock elements, which are found upstream of all heat shock genes (originally described by Pelham, Cell 30(1982):517-528), consist of up to seven repeats of a five-nucleotide module. Trimerization of heat shock factors was shown to be a prerequisite for high affinity binding to DNA in yeast (Sorger and Nelson, Cell 59(1989):807-813), and, in higher eukaryotes, heat shock factors trimerize upon heat stress, thereby increasing their affinity for heat shock elements.

In the last few years, over 40 new members of the fkh family have been cloned in species ranging from yeast to man. Some of these genes appear to play a central role in embryonic development. Examples are genes involved in notochord formation (*HNF*-*3β*; Ang and Rossant, Cell 78(1994):561-574; Weinstein et al., Cell 78(1994):575-588), in development of the neural axis in Xenopus laevis (*pintallavis*; Ruiz i Altaba and Jessel, Development 116(1992):81-93), in thymus development and hair growth in mice (*winged-helix nude*, the gene responsible for the nude phenotype in mice; Nehls et al., Nature 372(1994) 103-107), in brain development in rat (*Brain factor 1*; Tao and Lai, Neuron 8(1992):957-966) and in vulval cell fate in *Caenorhabditis elegans* (*lin-31*; Miller et al., Genes Dev. 7(1993):933-947). Two genes of the family have been shown to play a direct role in tumorigenesis. The chromosomal translocation involved in alveolar rhabdomyosarcoma results in a fusion between the genes coding for PAX3, a transcriptional regulator during early neuromuscular differentiation, and ALV, a member of the fork head family (Shapiro et al., Cancer Res. 53(1993):5108-5112). The oncogenic potential of the resulting chimeric protein suggests that transcriptional deregulation is the mechanism underlying the tumor development. Another winged-helix protein, encoded by the retroviral oncogene *qin*, found in the genome of the avian sarcoma virus 31, induces oncogenic transformation in cell culture and causes tumors in chickens (Li and Vogt, Proc. Natl. Acad. Sci. USA 90(1993):4490-4494).

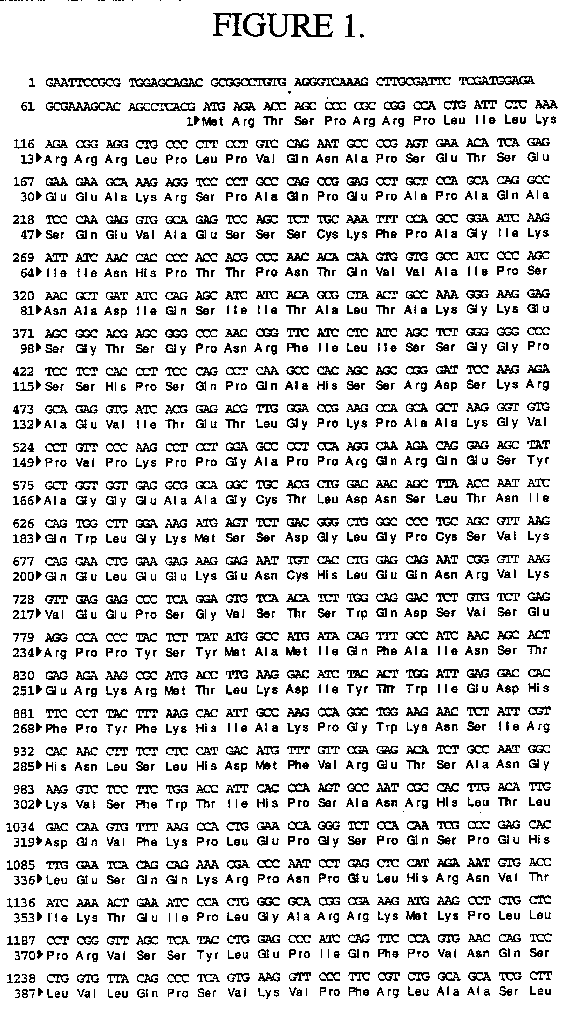

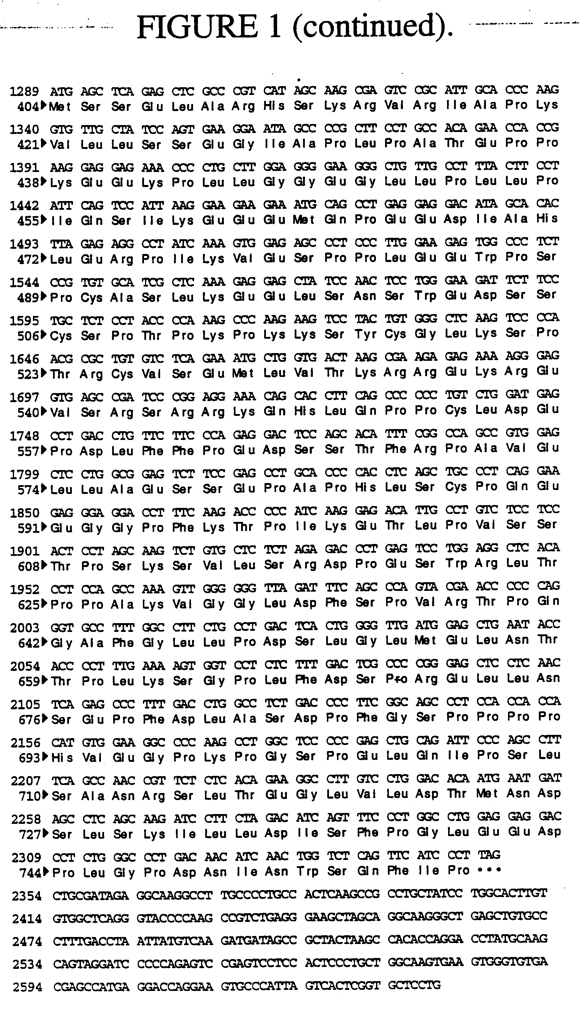

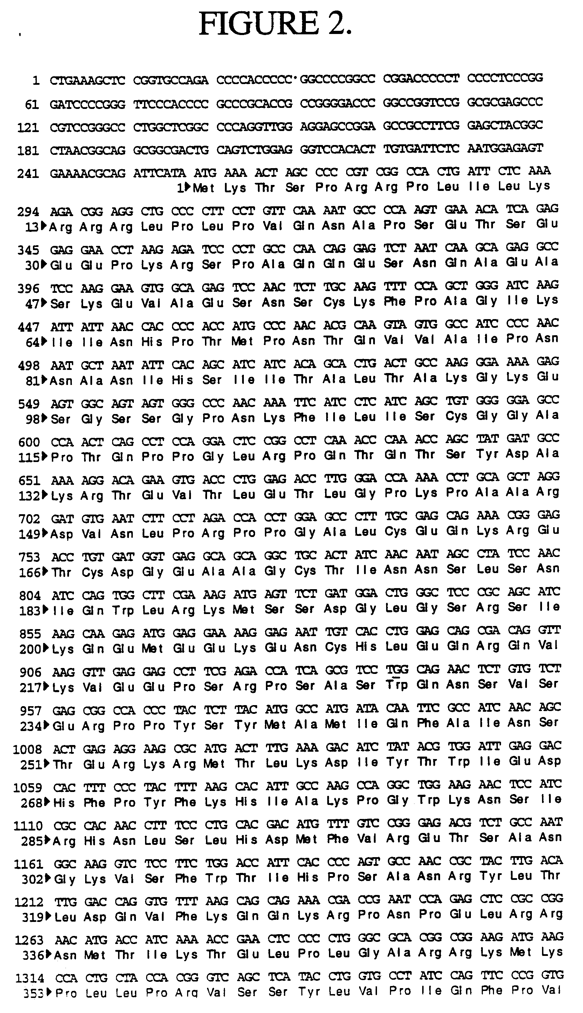

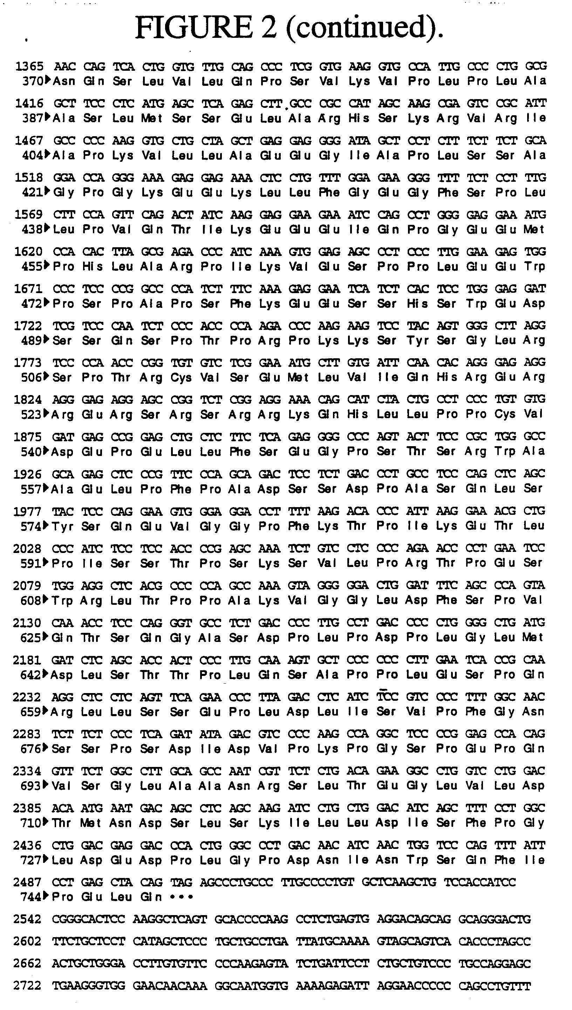

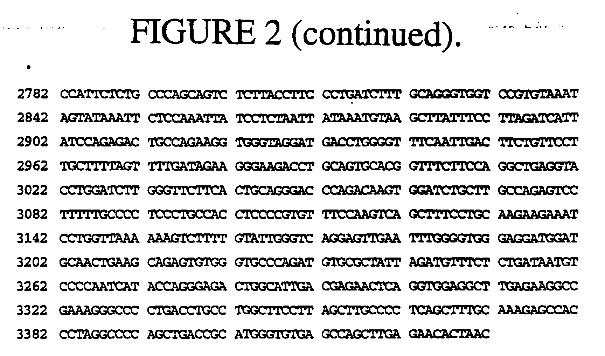

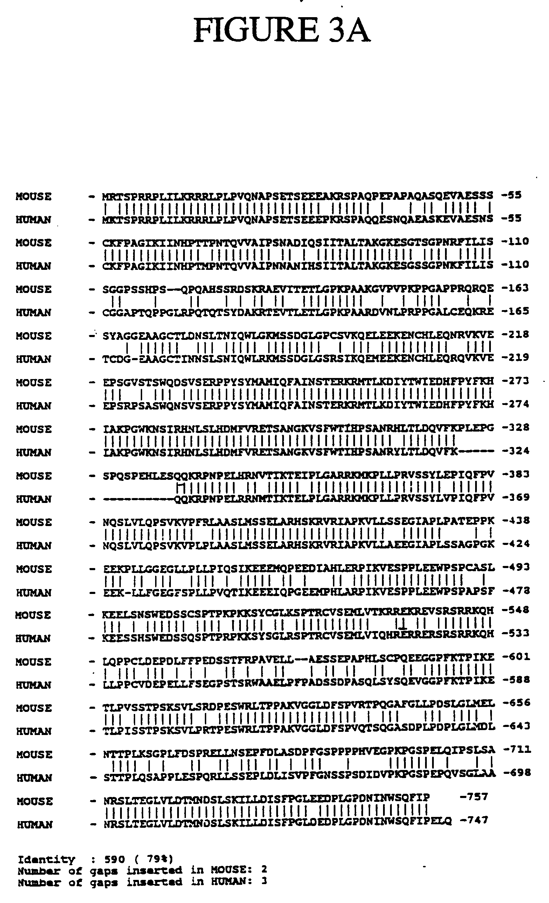

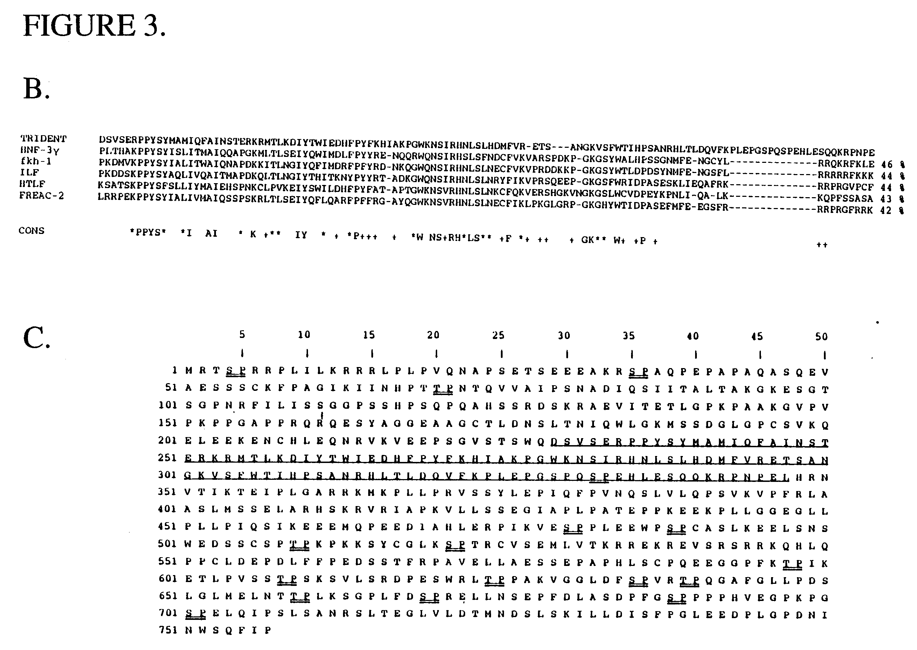

The present invention identifies new members of the fork head family. These were first identified by PCR reactions on mouse thymus cDNA using degenerate primers corresponding to conserved amino acids in the fork head DNA binding domain of HNF (see experimental part). In addition to PCR products encoding HNF-3g we found a sequence with low but significant homology (45% amino acid identity) to the fork head consensus domain. Using this 153 bp product as a probe we screened a mouse EL-4 thymoma cDNA library and isolated a clone with a 2673 nt insert containing a single large open reading frame of 2271 nt encoding 757 amino acids (figure 1). Subsequently, low-stringency screening of a cDNA library from a human leukemic cell line resulted in the isolation of a 3455 nt human cDNA clone with a single large open reading frame of 2241 nt encoding 747 amino acids, a 5' untranslated region of 257 nt and a 3' untranslated region of 957 nt (figure 2). The predicted amino acid sequences of the murine and human proteins are depicted aligned with each other in figure 3a, showing 79% amino acid identity. Figure 3b shows an alignment of the fkh DNA-binding motif of the murine protein with that of the five most closely related members of the fork head family, showing identities of around 45%.

Thus, the present invention discloses nucleic acid molecules encoding a new distant member of the winged-helix family, with relatively low sequence homology to the other family members, that is characterized by its DNA binding domain. The cloned gene was named *trident* and showed, apart from low homology to other members of the fkh family, a high match to the partial-length cDNA encoding the putative M-phase phosphoprotein MPP2 (*supra*). Comparison of the predicted TRIDENT amino acid sequence to known amino acid sequences revealed that its 3' end was identical to the predicted 221 amino acid sequence of the partial-length cDNA fragment of MPP2 (Westendorf et al., Proc. Natl. Acad. Sci. USA 91(1994):714-718). The C-terminus of TRIDENT contains several potential phosphorylation sites (see figure 3c). The central motif of the MPM2 binding site (T/S)P, as determined by Westendorf et al. (Proc. Natl. Acad. Sci. USA 91(1994) 714-718), was found 17 times in the TRIDENT sequence. Hence, we cloned the complete nucleic acid molecules encoding mouse and human TRIDENT/MPP2, that have only partially been predicted for the human protein before.

In one embodiment of the invention novel nucleic acid molecules can be constructed that encode a functional TRIDENT fragment or derivative. A functional TRIDENT fragment or derivative is characterized by its retaining of at least one property of the complete TRIDENT polypeptide. This includes, but is not restricted to, DNA binding, transactivation, and antigenicity (*infra*). Said novel nucleic acid molecules can be produced either by conventional cloning strategies known in the art, or by random mutagenesis techniques, including but not restricted to DNA shuffling (Stemmer, Proc. Natl. Acad. Sci. USA 91(1994):10747).

In another embodiment of the invention a nucleic acid molecule encoding TRIDENT or a functional fragment or derivative thereof can be cloned into an expression vector. Many prokaryotic and eukaryotic expression vectors useful for this aspect of the invention are known in the art. In the experimental part an example is shown where the complete human *trident* gene is cloned into expression construct pcDNAI for eukaryotic expression driven by a CMV promoter. Said expression constructs can be used to produce the TRIDENT protein or functional fragment or derivative thereof *in vitro* or intracellularly (after transfection of the expression construct). One of the uses of the produced polypeptide according to the present invention is to stimulate the transcription of genes regulated by a TRIDENT-responsive promoter. Said genes may either be naturally regulated by TRIDENT, or be cloned downstream from a TRIDENT-responsive promoter. The experimental part shows that human TRIDENT expressed after transfection of pcDNAI-h*trident* into B cells stimulates the expression of a marker gene cloned downstream from a TRIDENT-responsive promoter element. In yet another embodiment of the invention the TRIDENT protein or functional fragment or derivative thereof is used to identify and isolate by its binding genes that are regulated by a TRIDENT-responsive promoter.

In one aspect of the invention the functional TRIDENT fragments or derivatives retain wild-type TRIDENT DNA binding properties, but have altered transcription-stimulating activity. Polypeptides according to this aspect of the invention can be used, like the wild-type TRIDENT protein, to identify and isolate, through their binding to specific upstream DNA sequences, genes that are regulated by a TRIDENT-responsive promoter. In addition, said novel polypeptides can be used to compete with the wild-type TRIDENT protein for DNA binding. Expression of novel nucleic acid molecules that encode polypeptides that can bind to a TRIDENT-responsive promoter but that do not have transcription enhancing activity can be used to interfere with the expression of genes that are regulated by a TRIDENT-responsive promoter. Expression of novel nucleic acid molecules that encode proteins that can bind to a TRIDENT-responsive promoter and exhibit increased transcription enhancing activity as compared to wild-type TRIDENT can be used to augment the expression of genes that are regulated by a TRIDENT-responsive promoter.

In another aspect of the invention the functional TRIDENT fragments or derivatives represent a transactivation domain that can be operably fused to a DNA binding domain from a different polypeptide, for the regulation of expression of open reading frames that are operably linked to a binding site for said DNA binding domain. Important examples of DNA binding domains useful for this aspect of the invention include, but are not restricted to, the *lac* repressor DNA binding domain and the tetracycline repressor DNA binding domain. Said repressor DNA binding domains have been fused to exogenous transactivation polypeptides and the resulting fusion molecules exhibited transactivation through DNA binding.

As is clearly understood from the above, expression constructs encoding TRIDENT or fragments or mutant derivatives thereof can be used to stimulate or inhibit natural intracellular functions of TRIDENT or TRIDENT-related proteins. At present, these functions are unknown. However, the cell-cycle regulated expression of TRIDENT (*infra*) suggests a role for the protein in the progression of cells through the cell-cycle. Furthermore, preliminary results on *trident*-knockout mice that we have generated indicate that a null-mutation is lethal during embryogenesis. Thus, in one aspect of the invention said expression constructs are used to interfere with the cell-cycle.

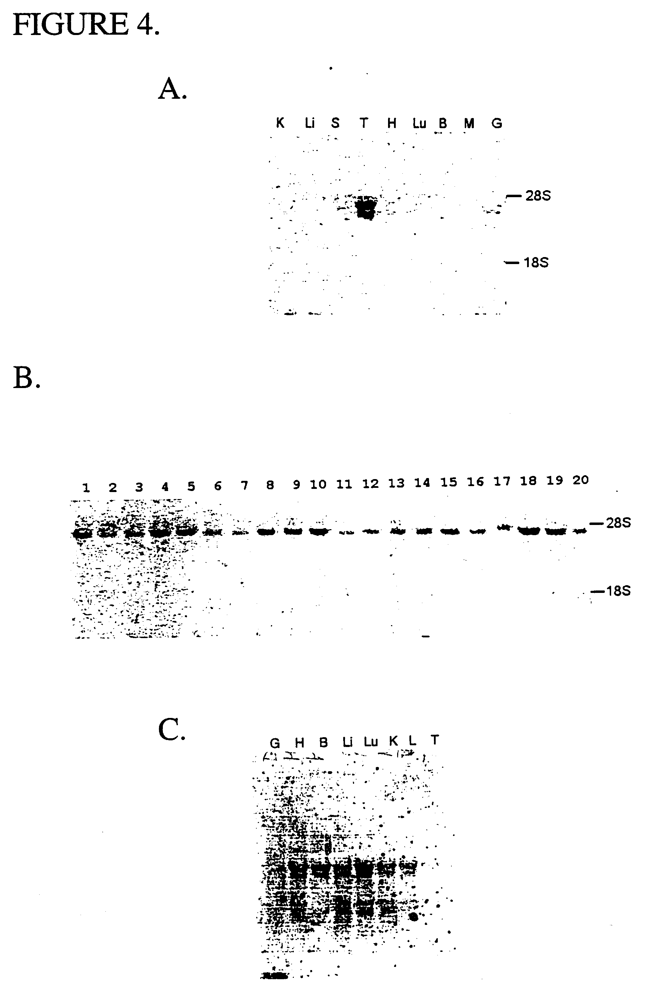

The expression patterns of the *trident* genomic genes in mouse and human cells were determined as follows. Northern blot analysis (see experimental part) of total RNA derived from various mouse tissues from a six week old animal demonstrated expression which appeared uniquely in thymus (figure 4a), suggesting T-cell specific expression of *trident*. Next, total RNA from twenty human cell lines was analyzed for *trident* expression by Northern hybridization (figure 4b). Unexpectedly, a single band of approximately 3.5 kb could be detected in all lanes representing cell lines ranging from B and T lymphoid, myeloid and erythroid to several carcinoma cell lines. By analyzing RNA from eight tissues from a day E14 mouse embryo, expression was detected in most tissues including heart, brain, liver and lung (figure 4c), in contrast with the thymus-restricted expression observed in the adult animal. These unexpected findings prompted us to look further into *trident* expression in cycling cells.

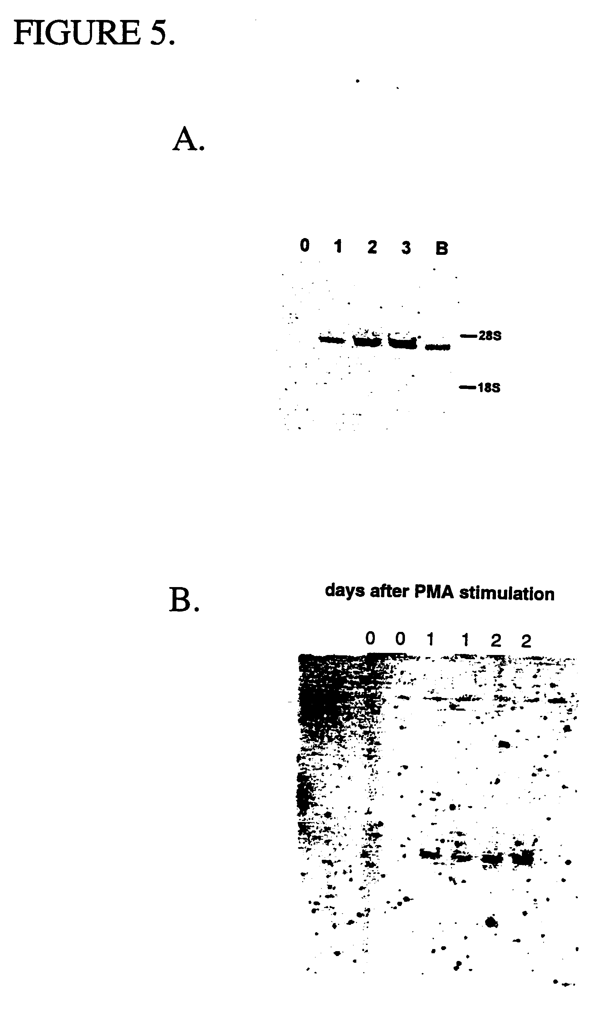

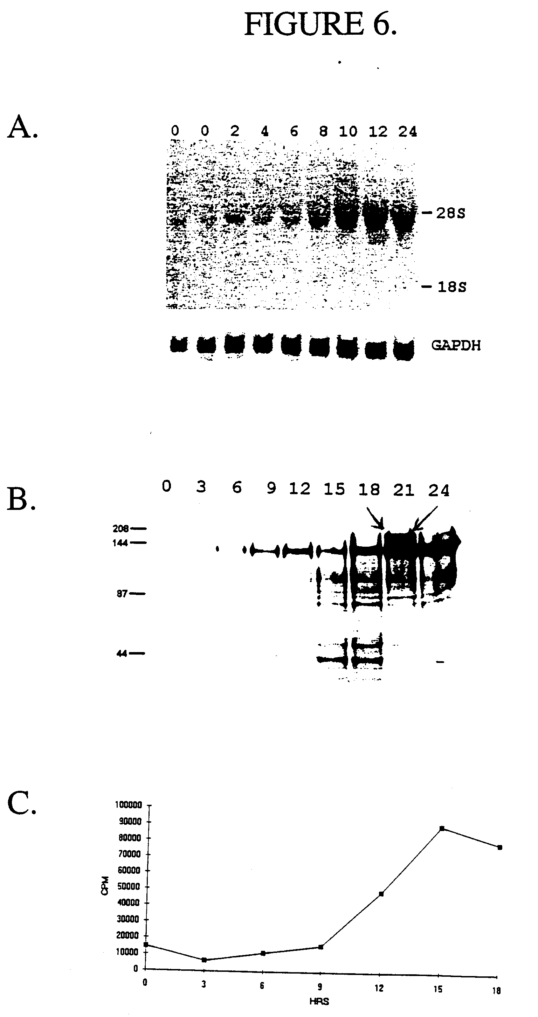

We used three model systems to test *trident* expression after stimulation of cells to enter cell-cycle (see experimental part). In the first model, human peripheral blood lymphocytes (PBL), which are essentially G₀ cells, were isolated from the blood of normal donors and stimulated with the T cell mitogen phytohaemagglutinin (PHA). On a Northern blot, no expression was detected in total RNA isolated from these cells on day 0 (figure 5a). *Trident* expression was induced within a day after PHA stimulation and reached its maximum on day 2. In the second model, a comparable expression pattern could be detected on a Northern blot made from B lymphocytes derived from a patient suffering from chronic lymphatic leukemia (CLL) stimulated with a phorbol ester (figure 5b). In the third assay, the rat fibroblast cell line Rat-1 was used. Cells were brought into G₀ by contact inhibition and serum starvation, and subsequently restimulated to enter cell cycle. Analysis of RNA isolated from samples taken every two hours showed a sharp rise in *trident* expression between 8 and 10 hours after stimulation (figure 6a). To confirm this observation at the protein level we analyzed protein samples of synchronized Rat-1 cells by Western blotting, using an anti-TRIDENT MoAb that we raised using a recombinant histidine-tagged human TRIDENT-fragment fusion protein (see experimental part). As is shown in figure 6b, a rise in the amount of TRIDENT protein followed the increase in *trident* mRNA levels around 9 hours after restimulation. This timepoint correlated with the initiation of the S-phase as was shown in a ³H-thymidine incorporation assay (figure 6c). Thus, *trident* is a novel member of the fork head family of transcription factors, that unexpectedly exhibited a cell-cycle dependent expression pattern. The initiation of the production of the TRIDENT protein coincides with that of S-phase, and the molecule presumably becomes phosphorylated upon entry into M-phase. This can be concluded from the identification of potential phosphorylation sites (*supra*), from the appearence of a protein with reduced mobility 21 hours after stimulation of synchronized Rat-1 cells (see figure 6b), and from the reactivity of MPP2 with the MPM2 MoAb (Westendorf et al. (1994). Proc. Natl. Acad. Sci. USA 91:714-718). Cell-cycle dependent expression has not been reported before for any known member of the fkh family. As a matter of fact, cell-cycle dependent expression is at all a very rare feature among transcription factors. It is, therefore, a very important characteristic of the novel member of the fkh family disclosed in the present invention.

In one embodiment of the invention the TRIDENT protein or a functional fragment or derivative thereof is used as an antigenic epitope to produce specific antibodies directed against the TRIDENT protein or parts thereof (see experimental part). The present invention provides monoclonal antibodies (MoAb) that specifically interact with TRIDENT protein. A MoAb according to the invention was designated 3C12A and was shown to interact with TRIDENT from several species, including at least human, mouse and rat, and to be useful for detection of TRIDENT protein in ELISA and Western analysis as well as in cells transfected with a *trident* expression construct (experimental part). In a further embodiment of the invention said antibodies are used for the detection of cells engaged in cell cycle. An important example of this aspect of the present invention is the use of said antibodies as a diagnostic tool for the detection of cycling malignant cells among normal cells that exhibit a much slower proliferation. In yet a further embodiment of the invention antibodies according to the invention are part of a kit that also comprises at least one other component that enables or supports the use of said antibodies for the purposes of the invention. Said components include, but are not restricted to, reaction buffer (components), a control peptide, a labelled secondary antibody, and the like. Those skilled in the art will be able to exploit antibodies or kits according to the invention in various other applications where cycling cells have to be discriminated from resting cells without departing from the present invention.

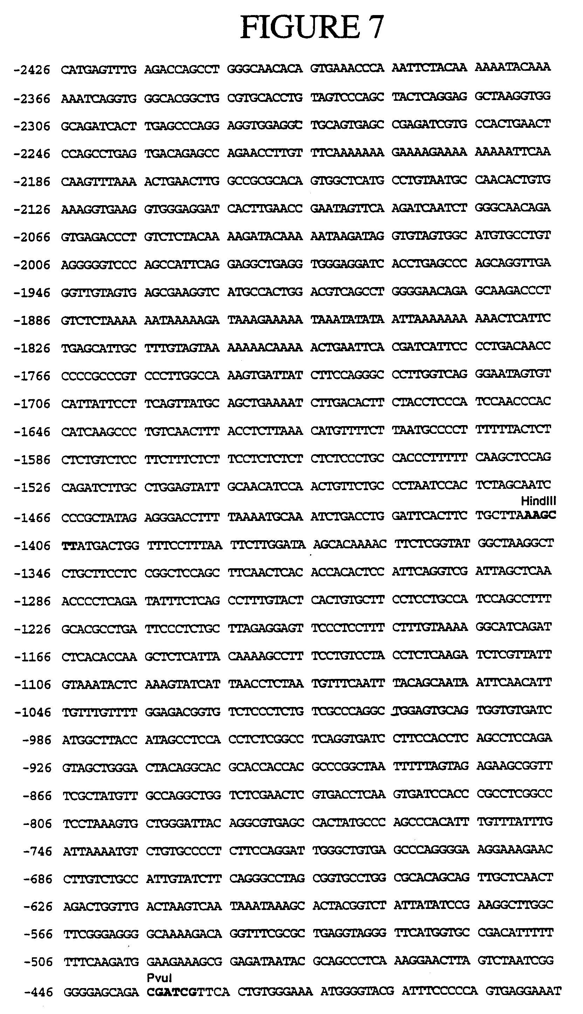

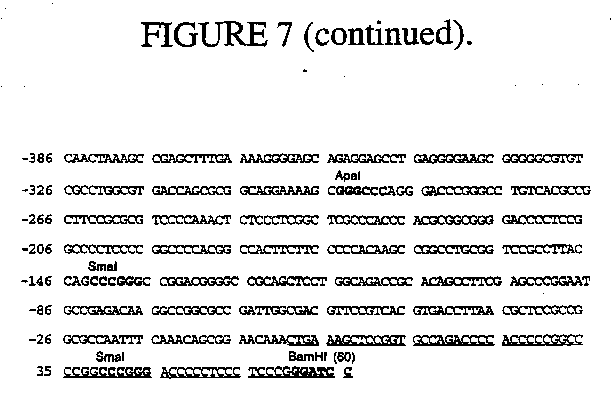

The human *trident* cDNA was used to isolate the complete human genomic *trident* gene including more than 10 kb upstream nucleotides (see experimental part). Subcloned upstream fragments were sequenced. Figure 7 shows the nucleotide sequence of a 2.5 kb upstream nucleic acid fragment. This fragment reaches until the BamHI site that is also present at position 60-65 in the human *trident* cDNA shown in figure 2. Thus, the last 65 nt in this sequence represent untranslated upstream sequences of the human *trident* cDNA.

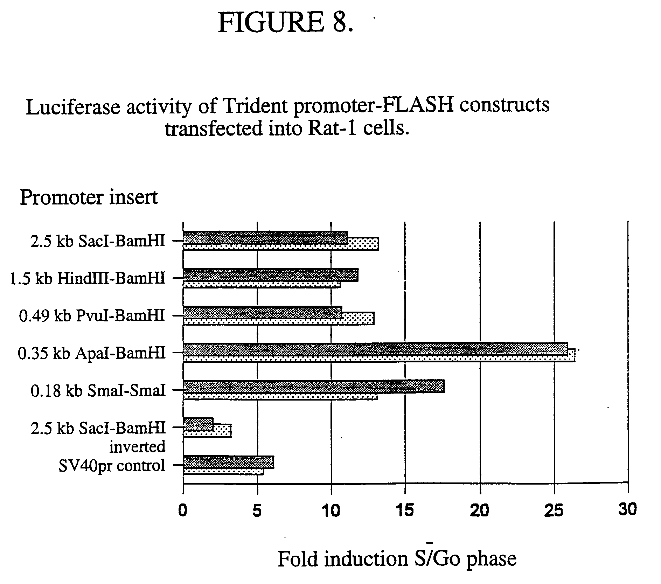

To locate the *trident* promoter within the upstream region, several deletion subclones were tested for their activity on a heterologous reporter gene encoding firefly luciferase in dividing and non-dividing cells (see figure 8 and the experimental part). Specific transactivation was measured in dividing cells during S-phase with all constructs. The *trident* promoter region cloned in the reverse orientation was inactive. The strongest transactivation was observed with a 353 nt ApaI-BamHI fragment containing 290 nt upstream sequences (see figure 7). Thus, the *trident* promoter, functionally characterized by its S-phase dependent transactivation, is located within the DNA sequence ranging from nt -290 to nt -1 from the first exon. This nucleic acid molecule contains one or more functional elements that are responsible for said S-phase dependent transactivation. Said functional elements could represent binding sites for one or more transactivating molecules. To obtain more insight in the nature of said functional elements the promoter region was analysed for the presence of possible binding sites for known transcription factors (see experimental part). None of the identified proteins with potential binding sites between positions -290 and -1 are known to have a cell-cycle dependent expression pattern. This makes a role for these proteins in the S-phase induced expression pattern of trident unlikely. The complete 2.5 kb upstream fragment was found to contain a single binding site for members of the transcription factor family E2F. The proteins of the E2F family, especially E2F-1, play a key role in cell-cycle regulation. E2F binding sites are crucial for transcriptional activation of genes that regulate S-phase entry (Müller, Trends Genetics 11(1995):173-178) and E2F-1, as a single factor, is capable of driving cell-cycle progression through G1 into S-phase upon ectopic expression in quiescent cells (Johnson, et al, Proc. Natl. Acad. Sci. USA 91(1994):12823-12827). Because E2F binding sites are absent from the 290 nt promoter fragment, a role for E2F proteins in the cell-cycle dependent transactivation of *trident* is very unlikely. This was confirmed by co-transfection of an E2F expresssion plasmid and the luciferase reporter construct carrying the 2.5 kb insert (see experimental part). The co-transfection did not influence the S-phase specific luciferase expression. Hence, the *trident* promoter according to the present invention is a novel nucleic acid molecule capable of transactivating the expression of nucleic acid molecules linked down-stream from said promoter upon binding of as yet unknown molecules that are present in cells during S-phase of the cell cycle.

In a further embodiment of the present invention a nucleic acid molecule encompassing a functional *trident* promoter characterized *supra* is used to drive the expression of genes cloned downstream of said nucleic acid molecule. This results in an expression pattern of said genes analogous to that of *trident*. This aspect of the invention is of particular importance for application in cases where expression of genes is desired in cycling cells only. Introduction of DNA constructs with said promoter and genes is achieved by numerous administration methods known in the art. Said methods include, but are not limited to, direct injection of naked DNA constructs, bombardment with gold particles loaded with said constructs, and macromolecule mediated gene transfer using, e.g., liposomes, biopolymers, and the like. In addition, gene delivery vehicles derived from viruses may be used, including but not limited to adenoviruses, retroviruses, and adeno associated viruses. Below some examples of applications of this aspect of the invention are given. Those skilled in the art will be able to apply the present invention in other situations where expression of genes is desired in cycling cells only and to use gene delivery methods or vehicles other than those exemplified herein without departing from the invention.

One very important application of the invention is in the treatment of tumors by transfer of genes that kill cells in which the introduced gene is expressed. Unlike normal tissues, tumors contain a relatively high fraction of dividing cells. This difference is already exploited in current standard gene therapeutic approaches for the treatment of cancer. For example, recombinant adenovirus vectors harboring the Herpes Simplex Virus-thymidine kinase (HSV-tk) gene are administered to brain tumors. Subsequently administered ganciclovir, a non-toxic nucleotide analogue, is phosphorylated by HSV-tk into a toxic compound. Phosphorylated ganciclovir is incorporated into replicating DNA chains where it acts as a chain-terminator. The interference with DNA replication implies that this procedure is mainly toxic to dividing cells. However, adenovirus vectors which result in transgene expression in dividing tumor cells and not in the surrounding non-dividing normal tissues provide an important improvement over current procedures, in particular when expression of the transgene is also to some extent toxic to normal tissues. Thus, expression of the transgene present in an adenovirus vector under direction of the *trident* promoter is also provided by the present invention. Therapeutic genes useful for this aspect of the invention include but are not limited to HSV-tk and other prodrug activating enzymes, tumor suppressor genes such as p53 and cytokine genes. An example of the latter is the use of the genes encoding interleukin-1 (Apte et al., In: Cytokine-induced tumor immunogenicity (1994) Acad. Press, London, pp.97-112) or interleukin-3 (McBride et al., Folia Biologica (Praha) 40(1993):62; Pulaski et al., Cancer Res. 53(1993):2112) to elicit an anti-tumor immune response, where high local concentrations are required for anti-tumor acitivity, but high systemic concentrations have severe adverse side effects.

In yet another embodiment of the invention, the *trident* promoter sequence is used to regulate cell-cycle dependent replication of the viral vector itself. In the most preferred embodiment of this aspect of the invention the viral vector is derived from an adenovirus. However, skilled artisans will be able to apply the teachings of this invention to vectors derived from other viruses, including but not restricted to DNA tumor viruses such as polyomaviruses or papillomaviruses. Application of replicating adenovirus vectors for *in vivo* treatment of cancer is favorable, as viruses produced in tumor cells after virus administration will spread to neighbouring cells, thereby ensuring a better penetration of the tumor. As the adenovirus DNA will replicate in the target cells, also higher expression of the therapeutic gene is achieved. Replicating, helper independent viruses have already been constructed and clinically applied (Berkner K.L. Curr. Topics in Microbiol. Immunol. 158(1992):39; Tacket et al. Vaccine 10(1992):673). Those viruses necessarily contain and express the E1 region, either in its 'natural position' (Berkner K.L. Curr. Topics in Microbiol. Immunol. 158(1992):39; see also e.g. Haj-Ahmad and Graham, J. Virol. 57(1986):267) or cloned and expressed elsewhere in the adenovirus genome (Levrero et al. Gene 101(1991):195). However, when a therapeutic gene encoding a toxic substance is used, replication of the recombinant adenovirus and expression of the therapeutic gene should be confined to the tumor tissue. In addition, when helper independent adenovirus vectors are used, not only potential toxic effects of the therapeutic gene should be circumvented but also toxicity associated with adenovirus production itself (adenovirus replication is lytic to cells). In order to achieve this, genes that are essential for replication of the adenovirus, such as e.g. the E1 gene, could be placed under the control of a tissue specific promoter. However, in most cases the tumor is derived from its surrounding tissue. Consequently, in many cases genes that are active in the tumor are also active in the surrounding tissue. Therefore, tissue specific promoters are less preferred to control replication of the adenoviral vector. In contrast, a much better approach is to drive the expression of at least one of the viral genes that are essential for replication by a cell-cycle dependent promoter. In one embodiment of the invention the adenovirus E1 gene is placed under the direction of the *trident* promoter. This limits replication of adenovirus vectors according to the invention to dividing cells, i.e. the tumor cells. If the tumor cells are replicating, the vector replicates and spreads to neighbouring cells, and this will iterate until the infected cells are killed by the action of the introduced therapeutic nucleic acid molecules thus blocking further virus spreading. If a non-replicating tumor cell or a normal tissue cell becomes infected, however, then no further replication will occur. Alternatively, other adenovirus genes are placed under the control of the *trident* promoter. Such genes are E2 (in particular E2A) and E4, as they are involved in replication of the adenoviral DNA as well. Of course, also more than one promoter of adenovirus genes may be replaced by the *trident* counterpart.

For the generation of replication competent recombinant adenoviruses according to the present invention a therapeutic gene (e.g. HSV-tk) is inserted either in the E3 region (see Berkner K.L., Curr. Topics in Microbiol. Immunol. 158(1992);39), in the E4 region or in the region between E4 and the right ITR (Saito et al., J. Virol. 54(1985):711; Saito et al., J. Virol. 58(1986):554). Insertion in the E3 region is preferred, because (i) the E3 region is dispensable for replication of the virus; (ii) viruses that do contain E1a sequences inserted at ectotopic postitions in the virus genome display only moderate expression (Levrero et al. Gene 101(1991):195) and proved to have no advantage over replication defective viruses; and (iii) similar viruses, harboring a gene encoding hepatitis B surface antigen, have already been used in clinical studies without any toxic side effects (Tacket et al. Vaccine 10(1992):673).

Yet another example of this application of the invention is expression of genes in replicating endothelial cells as a treatment for restenosis. A vector expressing a therapeutic gene is delivered *in situ* to the blood vessel wall by means of e.g. a catheter. An example of a gene used for treatment of restenosis is nitric oxide synthase (NOS; Janssens et al., Circulation 92(1995):2398; Leyen et al., Proc. Natl. Acad. Sci. USA 92(1995):1137). In this case also expression in non-dividing cells is undesired. Thus, expression of the therapeutic gene driven by a promoter according to the present invention provides an important improvement of such a procedure.

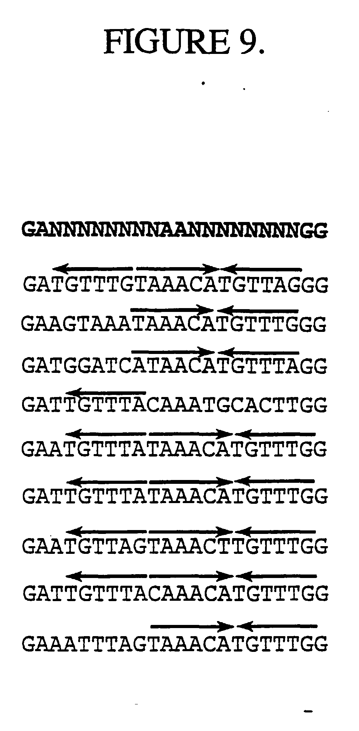

The present invention furthermore provides nucleic acid molecules that specifically interact with the DNA binding domain of TRIDENT. In order to identify the DNA binding specificity of TRIDENT, binding site selection was carried cut using a recombinant protein consisting of the mouse TRIDENT forkhead domain fused with maltose binding protein (MBP) (see experimental part). The chimeric protein was incubated with a pool of random oligonucleotides and sequences binding to the protein were isolated in a gel shift assay. Purified DNA from the shifted band was amplified using PCR and labeled for the next round of selection. After six rounds the oligonucleotides were subcloned and sequenced (see figure 9 for representative examples). Analysis of the selected sequences resulted in a consensus site (T/C)AAACA, in agreement with findings for other members of the family (Pierrou et al., EMBO J. 13(1994)5002-5012; Cardinaux et al., J. Biol. Chem. 269(1994):32947). This confirmed that *trident* encodes a functional fork-head type DNA binding domain. In contrast to findings for other members of the fkh-family, however, in 22 out of 36 sequenced motifs the binding site was found two or three times, indicative of di- or trimerization of the protein. The repeats of these motifs within one selected oligo were always in alternating orientation and with a fixed spacing as is indicated by the arrows in figure 9. Multiple repeats of the consensus site in alternating orientation has not been reported for any member of the fkh family. Thus, *trident* can be further discriminated from the known fkh-proteins by the unique structure of its binding site. The binding site pattern is very suggestive for trimerization of TRIDENT and reflects the organization of heat shock elements, to which heat shock factors bind in multimers (*supra*). For this, heat shock factors contain two well-defined regions involved in DNA binding and multimerization, respectively. In the TRIDENT protein, however, these two regions would both be located in the winged-helix domain, since the binding site selection experiments were performed using a recombinant protein of only that region.

To test whether binding of TRIDENT to its DNA binding site according to the present invention leads to functional transactivation of genes cloned downstream from said binding site, an expression plasmid encoding human TRIDENT was cotransfected into a B cell line with chloramphenicol acetyltransferase (CAT) expression constructs with oligonucleotide inserts containing the TAAACA TRIDENT DNA binding motif (see experimental part). Constructs with inserted TRIDENT binding sites exhibited significantly increased CAT activities after cotransfection with the TRIDENT expression construct as compared to the CAT expression constructs alone. This shows that (i) the human *trident* cDNA clone encodes a functional transcription factor, and (ii) the TAAACA binding site functions as a functional upstream transactivation binding site for human *trident* encoded protein. Hence, as already indicated above, the present invention provides TRIDENT-responsive promoter elements that are cloned upstream of exogenous genes to provide said genes with a cell-cycle regulated expression pattern. In another embodiment of the invention said elements are introduced into cells to serve as a so-called binding motif decoys. In this aspect of the invention said elements bind endogenously produced TRIDENT, thereby interfering with its natural transactivating activity.

### Brief description of the drawings:

Figure 1 depicts the nucleotide sequence of the mouse *trident* cDNA insert of 2640 bases in clone 62 (excluding poly-A tail), and underneath a prediction of the amino acid sequence of the encoded mouse TRIDENT protein, by translation of the open reading frame using the universal genetic code and Gene Construction Kit™ software.

Figure 2 depicts the nucleotide sequence of the human *trident* cDNA insert of 3459 bases in clone HCDNATOT (excluding poly-A tail), and underneath a prediction of the amino acid sequence of the encoded human TRIDENT protein, by translation of the open reading frame using the universal genetic code and Gene Construction Kit™ software.

Figure 3 depicts alignments of and identification of functional domains in the predicted amino acid sequences of the murine and human TRIDENT proteins and other members of the fkh family. (A) The predicted amino acid sequences of the murine and human TRIDENT proteins are presented aligned with each other. Vertical lines between the sequences indicate amino acid identity, dashes in the sequence indicate introduced gaps for optimal alignment. (B). Alignment of the mouse TRIDENT DNA binding domain with five other members of the fkh family. The conserved amino acids are presented below (CONS). '*' and '+' indicate conservation in five and four of the six proteins, respectively. The percentage amino acid identity to mouse TRIDENT is shown for each of the sequences at the right side of the figure. References for the depicted sequences: TRIDENT, *infra*; HNF-3g, Lai et al., Genes Dev. 5(1991):416; fkh-1, Kaestner et al., Proc. Natl. Acad. Sci. USA 90(1993):7628; ILF, Li et al., Genomics 13(1992):665; HTLF, Li et al., Genomics 13(1992):658; FREAC-2, Hellqvist et al., J. Biol. Chem. 271(1996):4482. (C). The DNA binding domain (underlined) and potential MPM2 binding sites (double underlined) are shown for the murine TRIDENT protein.

Figure 4 depicts expression of *trident* RNA in mouse tissues and human cell lines. (A). Northern blot analysis of total RNA prepared from various tissues of a six week old mouse. Tissues: K, kidney; Li, liver; S, spleen; T, thymus; H, heart; Lu, lung; B, brain; M, muscle; G, gut. (B). Northern blot analysis of total RNA prepared from a panel of human cell lines. 1, REH; 2, NALM-6; 3, APD; 4, BJAB; 5, DAUDI; 6, U266; 7, ARH; 8, CEM; 9, H-9; 10, JURKAT; 11, PEER; 12, HL-60; 13, KG-1; 14, THP-1; 15, U937; 16, K562; 17, colon 320; 18, HeLa; 19, HEPG-2; 20, GLC-8. (C). Northern blot analysis of total RNA prepared from various tissues of a day E14 mouse embryo. Tissues: G, gut; H, heart; B, brain; Li, liver; Lu, lung; K, kidney; L, total leg; T, thymus. At the right-hand side of the panels the positions of the ribosomal RNA bands are indicated.

Figure 5 depicts expression of *trident* RNA in cycling human cells. (A). Northern blot analysis on total RNA prepared from human PBL. Cells were harvested at 0, 1, 2 and 3 days after PHA stimulation as indicated above each lane. Lane B contains control RNA derived from a B cell line. (B). Northern blot analysis on total RNA prepared from human CLL cells harvested at day 0, 1, and 2 after stimulation with PMA. Analysis at each time-point was performed in duplicate.

Figure 6 depicts expression of TRIDENT in stimulated Rat-1 fibroblasts. Rat-1 cells were synchronized in G₀ and subsequently stimulated to reenter cell-cycle. (A). Northern blot analysis for *trident* RNA on total Rat-1 cell RNA at the time-points indicated above each lane. GAPDH expression is shown as an internal loading control. The positions of the ribosomal RNA bands are indicated. (B). Western blot analysis of TRIDENT protein in synchronized Rat-1 cells at the time-points after restimulation indicated above each lane on a 8% SDS-PAGE gel. At the left-side of the panel the positions of molecular weight markers (kD) are indicated. The arrows at 21 hours after stimulation indicate the protein with reduced mobility, possibly representing phosphorylated TRIDENT. (C). The ³H-thymidine incorporation profile of Rat-1 cells after restimulation.

Figure 7 depicts the nucleotide sequence of a 2.5 kb upstream fragment of the human *trident* gene containing the promoter region. Relevant restriction sites used for the generation of subclones are indicated. Untranslated upstream sequences overlapping with the *trident* cDNA sequence of figure 2 are underlined.

Figure 8 depicts the S-phase induction of the *trident* upstream fragments. The relative luciferase activity in S-phase Rat-1 cells as compared to the activity in Go cells is given for pFLASH constructs with the indicated promoter inserts. Light and dark bars represent results from duplicate samples.

Figure 9 depicts the DNA binding motifs of the human TRIDENT protein sound by binding site selection. The top line shows the sequence of the random oligonucleotides used for the selection. The nine sequences shown represent the different isolated binding motifs. The arrows indicate the orientations of the (C/T)AAACA consensus sequence.
The invention will be described in further detail in the following experimental part.

### Example 1.

### Cloning of mouse and human trident cDNAs.

Total RNA was prepared from mouse thymus using RNAzol (Cinna-Biotecx) according to the manufacturer's instructions. Poly A⁺ RNA was isolated with oligo(dT) Dynabeads. cDNA was made using random hexamers according to the protocol of the Perkin Elmer Cetus coorporation. The degenerate primers 5'-AA(G/A)CC(A/T/C)CC(A/T/C)TA(A/T)TC(G/A/T/C)TA(T/C)AT-3' and 5'-(G/A)TG(C/T)GT(G/A)AT(G/A/T/C)GA(G/A)TTCTGCCA corresponding to conserved amino acids KPPYSY and WQNSIRH in the fork head DNA binding domain of HNF, respectively, were used in a PCR (94°C, 60°C, 72°C all for 1 min, 35 cycles) on 100 ng cDNA. Products of 100-200 nt were excised from an agarose gel, blunted using T4 DNA polymerase, cloned into pBluescriptSK and sequenced. A novel 153 nt sequence with low but significant homology to the fork head consensus domain was isolated and used as a probe to screen an EL-4 cDNA library in lZAP (Stratagene), that was a gift from K. Georgopoulos (Boston), under standard conditions (Sambrook et al., 1989). We isolated a clone with a 2673 bp insert containing a single large open reading frame of 757 amino acids encoding mouse *trident* (figure 1). A human cDNA library was made using the HPB-ALL cell line (ATCC). Total RNA was prepared using RNAzol (Cinna-Biotecx) according to the manufacturer's instructions. Poly A⁺ RNA was isolated with oligo(dT) Dynabeads. cDNA was made using a oligo(dT) primer (copy-kit, Invitrogen). BstX1 adaptors (Invitrogen) were ligated to both ends and DNA fragments larger than 2 kb were isolated by excision after electrophoresis through agarose gel. The cDNA was cloned into the BstX1 site of pcDNAI/AMP (Invitrogen) and transformed into E. coli XL-1 blue. 1x10⁵ individual colonies were produced. Subsequently, the mouse cDNA was used as a probe to screen the human HPB-ALL cDNA library under low stringency conditions (final wash 40°C, 2xSSC). This resulted in the isolation of a 3481 bp human cDNA clone with a single large open reading frame encoding 747 amino acids encoding human *trident* (figure 2).

### Example 2.

### Production of an anti-TRIDENT monoclonal antibody.

To facilitate the production of a monoclonal antibody, a human *trident* cDNA-fragment coding for amino acids 392-558 was subcloned into pET-21 (Novagen) by PCR to produce a Histidine-tagged TRIDENT fusion protein. After transformation into E. coli. BL21 bacteria the fusion protein was produced and purified in a single affinity chromatography step on a Nickel-column according to the manufacturer's instructions. The resulting recombinant protein was used to immunize six-week-old Balb/c mice (5 mg protein/mouse) by subcutaneous injection in Freund's complete adjuvant on day 1, followed by a similar injection in Freund's incomplete adjuvant on day 10, followed by 7 intraperitoneal injections at weekly intervals of 5 mg fusion protein in phosphate-buffered saline (PBS). Three days after the last injection, the spleen was isolated and 5x10⁷ splenocytes were PEG-fused with 2.5x10⁷ NS-1 cells (ATCC) and HAT-selected according to standard procedures (J. Immunol. Meth. 35(1980):1-21). Ten to 14 days after hybridoma fusion, supernatants were initially screened in an ELISA against the fusion protein. As a negative control, an irrelevant Histidine-tagged fusion protein produced according to the same procedures as the TRIDENT fusion protein was used. The ELISA was performed as follows. First, 0.1-0.5 mg fusion protein per well was coated overnight at room temperature onto 96-well, round-bottom polyvinyl plates in 50 mM sodium carbonate buffer pH 9.6. After three washes in PBS with 0.05% Tween-20, the plates were blocked for 1 hour at 37^{o}C with 0.1% bovine serum albumine in PBS. After three washes, 50 ml hybridoma supernatant was added and incubated for 2 hours at room temperature. After three more washes, the plates were incubated for 2 hours at room temperature with HRPO-conjugated rabbit-anti-mouse immunoglobulin reagent (1:300 in PBS+0.05% Tween-20+ 1% fetal calf serum; RAMPO, Dakopatts, Glostrup, Denmark). After three washes, the plates were incubated for 15-30 minutes at room temperature with 0.01% tetramethylbenzidine substrate in sodium acetate buffer pH 5.8 with H₂O₂. Finally, the reaction was stopped with H₂SO₄ and staining was measured at 450 nm. Positive supernatants were tested on COS-1 cells transfected with full-length TRIDENT expression constructs. To this end, COS-1 cells were trypsinized from petri dishes, washed in PBS, and incubated for 1 hour at 37^{o}C in RPMI containing 0.4 mg/ml DEAE/dextran (Pharmacia) and 1 mg/ml of the complete human *trident* cDNA in pcDNAI/AMP (see above). Cells were then washed once and plated in 96-well, flat-bottom culture plates at a concentration of 10⁴ per well. After 48 hours, the cells were washed once with PBS, fixed in the plates with 100% methanol for 20 minutes at room temperature, and stored under methanol at -20^{o}C. After washing the plates once with PBS, 50 ml hybridoma supernatant was added and incubated for 2 hours. The plates were washed once with PBS and 1 hour incubated at room temperature with HRPO-conjugated rabbit-anti-mouse immunoglobulin reagent (1:300 in PBS; RAMPO, Dakopatts, Glostrup, Denmark). After two washes in PBS, a color reaction was performed with 0.02% 9-amino-3-ethylcarbazol/0.1% hydrogenperoxide in 0.1 M sodium acetate, pH 4.8. Staining was detected using an inverted microscope. After several rounds of subcloning, a hybridoma was selected producing an antibody of the IgG1 subclass. This anti-TRIDENT monoclonal antibody designated 3C12A was found to be reactive in Western blotting and cell staining. Surprisingly, it reacted with the human, rat and mouse TRIDENT proteins.

### Example 3.

### Analysis of trident expression in mouse tissues and human cell lines.

A six-week-old mouse was sacrificed and kidney, liver, spleen, thymus, heart, lung, brain, muscle, and gut were individually dissected. Total RNA was isolated from these tissues with RNAzol (Cinna-Biotecx) according to the manufacturer's instructions. Fifteen µg RNA from each tissue was electrophoresed through 1% agarose gel containing 2% formaldehyde, and transferred to nitrocellulose according to standard procedures (Sambrook et al., 1989). The blot was hybridyzed with a ³²P-dCTP-labeled 668 bp EcoRI-PstI mouse *trident* cDNA fragment and washed with a final stringency of 0.2 x SSC, 68^{o}C. Exposure to a PhosporImager revealed strong *trident* expression in thymus and a faint signal in gut (figure 4a). Next, total RNA was isolated as above from a panel of 20 different human cell lines. All cell lines were obtained from the ATCC and were cultured in RPMI 1640 medium (Gibco, Paisley, U.K.) supplemented with 5% FCS and the antibiotics penicillin and streptomycin (Gibco, Paisley, U.K.) at 37^{o}C, 5% CO₂ in a 100% humidified atmosphere. Cell lines tested include: REH (pro-pre B cells), NALM-6 (pre B cells), APD (B cells), BJAB, DAUDI (blasts) U266, ARH (plasmacells), CEM (T cells), H-9, JURKAT (T cells), PEER (T cells), HL-60 (myeloid cells), KG-1 (myeloblasts), THP-1 (monocytes), U937 (monocytes), K562 (erythroid progenitors), colon 320 (colon carcinoma), HeLa (cervix carcinoma), HEPG-2 (liver carcinoma), and GLC-8 (lung carcinoma). Northern analysis was performed as above, using the human *trident* SmaI cDNA fragment from nt 75-2550 as a probe. Figure 4b shows that a single band of approximately 3.5 kb could be detected in all cell lines. Thus, *trident* expression was not restricted to T cells. In contrast, *trident* expression was detected in every cell type analyzed. Next, total RNA was isolated from tissues dissected from a mouse embryo at day-14 after gestation and examined for *trident* expression by Northern analysis as above. Expression was clearly detectable in heart, brain, liver, lung, kidney, thymus, and total leg tissue (figure 4c). In all mouse tissues, a smaller band of approximately 2.5 kb was found in addition to the 3.5 kb band also present in human cells. We contribute the smaller transcript to alternative splicing or polyadenylation.

### Analysis of trident expression in PHA-stimulated human PBL and phorbol ester-stimulated CLL.

Human peripheral blood lymphocytes (PBL) were isolated from heparinized total blood from a healthy donor by Ficoll-Isopaque centrifugation according to standard procedures. The cells were seeded 4x10⁴ cells per well in 96-well round-bottom plates and grown for three days in RPMI 1640 supplemented with 5% FCS, 2 mM glutamine, penicillin, streptomycin and 5 µg/ml PHA at 37^{o}C, 5% CO₂ in a 100% humidified atmosphere. At the initiation of the culture and at days 1, 2, and 3 after PHA stimulation, cells were harvested and total RNA was isolated using RNAzol as above. Northern analysis was performed as described for human cells in example 4 using 15 mg RNA per lane. As can be seen in figure 5a, *trident* expression was not detected in freshly harvested PBL at day 0, but was induced within a day after PHA stimulation and reached its maximum on day 2.

An almost monoclonal population of resting B cells was isolated from the peripheral blood of a patient with chronic lymphatic leukemia (CLL). The B cells were incited to enter cell-cycle as described for PBL, with PHA substituted by 5 ng/ml phorbol ester PMA. At day 0, and at days 1 and 2 after PMA stimulation, cells were harvested and total RNA was isolated using RNAzol as above. Northern analysis was performed as described above. As can be seen in figure 5b, *trident* expression was not detected in freshly harvested PBL at day 0, but was also in these cells induced within a day after stimulation.

### Example 5.

### Analysis of trident expression in cycling and non-cycling Rat-1 fibroblasts.

The rat fibroblast line Rat-1 (ATCC) was grown in Dulbecco's MEM with 10% FCS and penicillin and streptomycin at 37^{o}C, 10% CO₂ in a 100% humidified atmosphere. To bring the cells into G₀, they were grown to a confluent monolayer and synchronized in medium containing 0.5 % serum for 48 hrs. Next, they were stimulated to reenter cell-cycle by reseeding at low density and restimulation with medium containing 10 % fetal calf serum. Northern analysis of RNA isolated as described above from samples taken every two hours showed a sharp rise in *trident* expression between 8 and 10 hours after stimulation (figure 6a). This observation was confirmed at the protein level by analyzing protein samples of synchronized and restimulated Rat-1 cells (figure 6b). To this end, 3x10⁵ Rat-1 cells were plated on 10-cm dishes in medium containing 10% FCS. After an overnight incubation at 37^{o}C, 10% CO₂ in a 100% humidified atmosphere the medium was replaced with medium containing 0.5% FCS and cultured for 48 hours at 37^{o}C, 10% CO₂ in a 100% humidified atmosphere. Next, the medium was replaced by medium containing 10% FCS (t=0) and the cells were cultured as before. During a culture period of 24 hours, every 3 hours a sample was used for protein isolation. The cells were washed with PBS and resuspended in 30ml sample buffer (15 mmol/L Tris pH 6.8, 2.5% SDS, 20% glycine, 0.005% bromophenol blue, 4% b-mercaptoethanol). After sonification for 20 seconds and boiling for 5 minutes, the protein samples were separated on 8% SDS-PAGE acording to standard procedures (Laemmli, Nature 227(1970) 680). Gels were blotted to nitrocellulose membranes (Schleicher & Schuell, Keene, NH). The membranes were blocked with 5% milk in washing buffer (PBS with 0.1% Tween-20) for 2 hours, incubated with the MoAb 3C12A (1:1 diluted in 4% Protifar, with addition of 2% final concentration fetal calf serum) for 2 hours, washed for 15 minutes in washing buffer, and incubated with RAMPO (diluted 1:2000) for 2 hours. The specific antibody signals were detected with an enhanced chemiluminescence kit (ECL; Amersham, Buckinghamshire, UK) by exposure of an X-ray film. Nine hours after restimulation, a protein band of approximately 130 kD was detected by the anti-TRIDENT 3C12A MoAb. This apparent MW different from the predicted size of 85 kD is probably caused by anomalous running behaviour on SDS-PAGE, because it was also observed for *in vitro* translated TRIDENT that has not been modified posttranslationally (not shown). After 21 hours, a second molecule exhibiting a slower migration pattern was detected. Smaller bands at high TRIDENT concentrations are contributed to protein degradation.

To examine which stage of the cell-cycle the rise in *trident* expression coincided with, Rat-1 cells were plated on 24-well plates (10,000 cells/well) in medium containing 10% FCS. Next, the cells were synchronized and restimulated as described for the Western analysis. During a culture period of 18 hours, every 3 hours 1 µCi of ³H-thymidine was added to one of the wells and the cells were incubated for 1 hour. After this, the cells were washed three times with PBS, fixed with 1 ml methanol per well for 30 minutes, washed once with PBS again and then resuspended in 0.75 ml of 0.2 N NaOH. The cell suspension was added to 4 ml of Packard opti-fluor scintillation liquid and the c.p.m. representing the amount of incorporated ³H-thymidine was determined on a Packard 1900CA liquid scintillation counter. Figure 6c shows that ³H-thymidine incorporation was initiated 9 hours after restimulation of the cells, indicating that by that time the synchronized Rat-1 cells entered the S-phase of cell-cycle.

### Example 6.

### Analysis of the trident promoter region.

The human *trident* cDNA clone in pcDNAI/AMP (see example 1) was used to isolate an approximately 80 kb human genomic fragment inserted in a P1 plasmid (experiment carried out under contract at GenomeSystems, St. Louis, MO). This fragment contains the complete human *trident* gene and more than 10 kb upstream sequences. This DNA was digested with ApaI or SacI and subcloned into pBluescriptSK. The various subclones were partially sequenced using a universal primer against Bluescript vector sequences according to standard procedures. The identification of exon-intron boundaries allowed the construction of a partial map of the human *trident* gene. The coding sequences are divided over 8 exons encompassing approximately 35 kb genomic DNA. Sequences upstream of the coding region were determined on a 2.5 kb SacI-BamHI subclone in pFLASH (Mol. Cell. Endocrinol. 81(1991):81-94) under contract at BaseClear, Leiden, The Netherlands. The sequence of nucleotides - 2426 to - 1 relative to the predicted start of exon 1 (the first nucleotide of the cDNA shown in figure 2) is given in figure 7. Restriction enzyme digestion sites relevant for the construction of subclones are indicated.

The nucleic acid sequence of the 2.5 kb SacI-BamHI insert was analysed for the presence of binding motifs for known transcription factors. This was done according to the methods described by Quandt et al (Nucl. Acid. Res. 23(1995):4878) using the MatInspector software program. DNA binding proteins for which potential binding sites were identified are included in table 1.

**Table 1**

| Analysis of the 2.5 kb SacI-BamHI human *trident* promoter fragment for potential protein binding sites. (sequence analysed: positive strand nt - 2384 to -1; solution parameters: core similarity: 1.00, matrix similarity: ≥0.95) | |
|---|---|
| DNA binding protein | Nucleotide Position(s) |
| activator protein 1 | -619 |
| c-Myb | -641 |
| deltaEF1 | -2344; -1969; -944 |
| E2F | -543 |
| GATA-binding factor 1 | -2035; -2034; -1871; -1867; -1379; -483 |
| homeo domain factor Nkx-2.5 | -829 |
| Ikaros 2 | -923; -797 |
| MZF1 | -1908; -448; -365; -346; -220 |
| N-Myc | -50 |
| S8 | -1851 |
| sterol regulatory element-binding protein | -50 |
| upstream stimulating factor | -50; -49 |

To determine which potential protein binding sites play a role in the function of the *trident* promoter several different upstream fragments (0.18 kb SmaI, 0.35 kb ApaI-BamHI, 0.49 kb PvuI-BamHI, 1.5 kb HindIII-BamHI, and 2.5 kb SacI-BamHI) were made blunt-end and subcloned into the PvuII-site of the luciferase reporter construct pFLASH (Mol. Cell. Endocrinol. 81(1991):81-94). As controls, in one construct the 2.5 kb upstream fragment was inserted in the reverse orientation and in another construct a SV40 promoter fragment was inserted. Each construct was transfected into Rat-1 cells by electroporation using standard procedures. Next, the samples were split in two and subjected to serum-starvation (*supra*). After 24 hours, one sample of each set was stimulated to enter cell-cycle by serum addition. Sixteen hours later, a small aliquot was taken of all samples to determine the cell-cycle state of the cells by FACS analysis. This revealed that virtually all serum-deprived cells were in Go, whereas cells stimulated to enter cell-cycle were in late-S to M-phases of the cell-cycle (not shown). The remaining cells were lysed in 25 mM Tris-phosphate buffer pH 7.8, containing 8 mM MgCl₂, 1 mM DTT, 15% glycerol, and 1% Triton X-100, and luciferase activity was measured after addition of 1 mM luciferin and 1 mM ATP. In Go, luciferase activities of all constructs were very low. Figure 8 depicts the relative activities of the promoter fragments in S-phase Rat-1 cells compared to those in Go-cells. As can be seen, the negative control construct carrying a SV40 promoter fragment exhibited 5 to 6-fold more luciferase activity in S-phase cells than in resting cells. This was not an unexpected finding, because in resting cells the availability of components of the expression machinary, such as transcription factors and ribosomes, may be limited. However, the *trident* promoter constructs showed a much more enhanced S-phase induction (approximately 12-fold). Most interestingly, the small ApaI-BamHI insert comprising 290 nt *trident* promoter region sequences induced luciferase expression in S-phase more than 25-fold. The control construct carrying an inverted *trident* promoter fragment was less active than the SV40-control.

None of the proteins with a potential binding site on the 290 nt *trident* promoter region (see table 1) are known to have an S-phase dependent expression or binding pattern. This makes a role for these factors in S-phase dependent induction of *trident* unlikely. The complete 2.5 kb SacI-BamHI fragment, however, contains a binding site for transcription factors of the E2F family (position -543). E2F binding sites are assumed crucial for transcriptional activation of genes that regulate S-phase entry. Althought these binding sites are absent from the promoter fragment with the strongest S-phase dependent transactivation activity, we performed the following control experiment: the pFLASH construct with the 2.5 kb promoter region inserted was co-transfected into Rat-1 cells together with an expression plasmid carrying the gene encoding the transcription factor E2F-1, a potent member of the E2F family. The S-phase induction of luciferase expression was measured as described *supra*. It was found to be similar with or without E2F-1. This shows that E2F-1 does not play a role in the functioning of the *trident* promoter.

### Example 7.

### Identification of the DNA binding specificity of TRIDENT.

In order to identify the DNA binding specificity of TRIDENT, binding site selection was carried out using a recombinant protein consisting of the murine TRIDENT forkhead domain fused with maltose binding protein (MBP). This fusion protein was made by subcloning the cDNA coding for the murine TRIDENT amino acids 207-344 into pIH902 (New England Biolabs), according to the manufacturer's instructions. Ten nanogram of the fusion protein was incubated with 0.2 ng of a pool of [g-³²P]ATP end-labeled random oligonucleotides in a buffer containing 10 mM Tris (pH 8.0), 5 mM MgCl₂, 50 mM NaCl, 0.1 mM EDTA, 5% glycerol and 0.1% Nonidet P-40 for 30 minutes at room temperature. The random oligo (sequence 5'-CTGGGTACCTCGAGTGAAGCTTGANNNNNNNNAANNNNNNNNGGGAATTCGGATC CGCGGTAAC-3') contained two fixed A residues in the middle of the random stretch to enhance binding based on the consensus binding sequence of other fork head proteins (Pierrou et al., 1994). Next, sequences binding to the protein were isolated in a gel shift assay on a 5% polyacrylamide gel run with 0.25 x TBE. Purified DNA from the shifted band was amplified using PCR (94°C, 60°C, and 72°C, each for 30 Sec; 35 cycles) with the primers 5'-CTGGGTACCTCGAGTGAAGCTTGA-3' and 5'-GTTACCGCGGATCCGAATTCCC-3' and labeled for a next round of binding selection as before. After six rounds the oligonucleotides were subcloned into pBluescript vector and 36 individual clones were sequenced. Nine representative sequences are depicted in figure 9. The Trident consensus binding site was determined essentially according to Blackwell and Weintraub (Science 250(1990):1104-1110) with modifications according to Van Dijk et al. (Proc. Natl. Acad. Sci. USA 90(1993):6061-6065). Analysis of the selected sequences resulted in a consensus site (T/C)AAACA, in agreement with findings for other members of the family (Pierrou et al., EMBO J. 13(1994)5002-5012; Cardinaux et al., J. Biol. Chem. 269(1994):32947). However, in 22 out of 36 sequenced motifs this site was found two or three times, indicative of di- or trimerization of the protein. The repeats of these motifs within one selected oligo were always in alternating orientation as is indicated by the arrows in figure 9.

### Example 8

### Transactivation of a reporter gene by TRIDENT binding to upstream TAAACA binding sites.

To test whether binding of TRIDENT to its DNA binding site leads to functional transactivation of genes cloned downstream from said binding site, three different oligonucleotides containing one, two, or three TAAACA TRIDENT binding sites in alternating order were cloned into vector pBLCAT2 (Luckow and Schultz, Nucleic Acids Res. 15(1987):5490) that contains the gene encoding chloramphenicol acetyltransferase (CAT) downstream from a minimal HSV-thymidine kinase promoter. pBLCAT2 without oligonucleotide insert served as a negative control. Full length human *trident* cDNA was cloned into pcDNAI (Invitrogen) to yield TRIDENT expression driven by the CMV promoter as described above. Ten microgram CAT reporter plasmid was transfected together with 1 microgram pcDNAI-h*trident* into 5.10⁶ AZUII B cells (J. Immunol. 136(1986):348) as described by Van de Wetering et al (EMBO J. 12(1993):3847). After 48 hours, cells were harvested and CAT activity was measured. To this end, cells were lysed in 10 mM Tris pH 7.5, 50 mM NaCl, 1 mM EDTA by freeze-thawing and lysates were cleared by centrifugation (10.000*g*, 5 minutes). CAT assay mixture was added to reach a final volume of 200 microliter per sample and concentrations of 100 mM Tris pH 7.5, 25 mM NaCl, 1.4% glycerol, 0.5 mM EDTA, 0.5 mg/ml Butyryl-CoA with 2 microCi ¹⁴C-chloramphenicol and reactions were allowed to proceed for 2 hours at 37^{o}C. Butyrylated chloramphenicol was extracted with xylene-pristane and the activity was measured in a liquid scintillation counter. CAT expression from the pBLCAT2 construct without oligonucleotide insert was elevated slightly by cotransfection with pcDNAI-h*trident* (1.4-fold) by a non-specific mechanism. Constructs with inserted TRIDENT binding sites, however, showed 3.3-5.2-fold increased CAT activity after cotransfection with the pcDNAI-h*trident* construct as compared to the CAT expression construct alone. This shows that (i) the human *trident* cDNA clone encodes a functional transcription factor, and (ii) the TAAACA binding site functions as a functional upstream transactivation binding site for human *trident* encoded protein.

## Claims

1. A recombinant or isolated nucleic acid molecule comprising a sequence derived from a gene encoding a transcription factor of the fork head family which factor is preferentially expressed in dividing cells or a functional part or derivative of said sequence derived from said gene.

2. A nucleic acid according to claim 1 comprising at least a functional part or derivative of the sequence depicted in figure 1 or 2.

3. A nucleic acid molecule according to claim 1 comprising at least a functional part of the promoter or a functional derivative thereof of the gene encoding the transcription activator.

4. A nucleic acid according to claim 3 comprising at least a functional part of the sequence depicted in figure 7.

5. A nucleic acid according to claim 3 or 4 further comprising a sequence encoding a protein of interest to be expressed in dividing cells, which sequence lies downstream from the functional promoter in functional alignment with said functional promoter.

6. An expression vector comprising a nucleic acid according to anyone of the aforegoing claims.

7. A cell comprising a nucleic acid according to anyone of claims 1-5 or an expression vector according to claim 6.

8. A method for expressing a protein of interest essentially exclusively in dividing cells, comprising providing said cells with a nucleic acid comprising a sequence encoding said protein under control of a functional promoter derived from the promoter sequence given in figure 7 and culturing said cells under conditions allowing for expression of said protein.

9. A transcription activator encoded by a nucleic acid according to any one of claims 1-3 or obtaianable by expressing a vector according to claim 6 or by culturing a cell according to claim 7.

10. A recombinant or isolated nucleic acid comprising a binding site for a transcription activator according to claim 9.

11. A nucleic acid molecule according to claim 10 containing at least two copies of the consensus site (T/C)AAACA.

12. A nucleic acid molecule according to claim 11, containing a multimer of said consensus site.

13. A nucleic acid molecule according to claim 11 or 12, wherein said consensus sites are organized in alternating orientations.

14. A nucleic acid molecule according to anyone of claims 11-13, wherein an open reading frame is located downstream from said consensus sites.

15. A polypeptide molecule with at least more than 50% homology, preferably more than 70%, particularly more than 90% homologyto the polypeptide molecule shown in figure 3a or 3b, or a functional fragment or derivative thereof.

16. A complex of a polypeptide molecule according to any of the claims 9 or 15 with a nucleic acid molecule according to anyone of claims 10-14.

17. The use of a complex according to claim 16 to identify and/or isolate said nucleic acid molecule.

18. A recombinant or isolated nucleic acid molecule with at least 50%, preferably 70%, in particular 90% homology to the nucleic acid molecule shown in figure 1 or the nucleic acid molecule shown in figure 2, or a fragment or derivative thereof.

19. An expression vector containing a nucleic acid molecule according to claim 18.

20. A cell transduced with an expression vector according to claim 19.

21. The use of a nucleic acid molecule according to claim 4 to drive the expression of said open reading frame dependent on the presence of a polypeptide according to anyone of the claims 9 or 15.

22. The use of a nucleic acid molecule according to anyone of claims 10-13 as a decoy for polypeptide molecules according to anyone of claims 9 or 15, for interfering with transactivation processes.

23. A vector according to claim 6 or 19, where said vector is derived from a virus, preferably from an adenovirus, an adeno associated virus, or a retrovirus .

24. A vector according to claim 23, whereby said open reading frame encodes one of the viral genes.

25. A vector according to claim 23 or 24, whereby said vector is helper independent.

26. A cell transduced with a vector according to anyone of the claims 23-25.

27. The use of a vector according to anyone of the claims 23-25 for cell-cycle dependent expression of said open reading frame.

28. The use of a vector according to claim 25 to regulate cell-cycle dependent replication of said vector.

29. A pharmaceutical composition containing a vector according to anyone of claims 23-25.

30. The use of a pharmaceutical composition according to claim 29 for the treatment of malignant diseases, whereby said pharmaceutical composition is directly injected into a tumor.

31. The use of a pharmaceutical composition according to claim 29 for the treatment of malignant diseases, whereby tumor cells are removed from a living body, subsequently treated *ex vivo* with said pharmaceutical composition, and finally are reinjected.

32. The use of a pharmaceutical composition according to claim 29 for the prevention of restenosis, whereby said pharmaceutical composition is delivered *in situ* to cells of the blood vessel wall.

33. An antibody directed against a polypeptide molecule according to any one of claims 9 or 15.

34. An antibody directed against an epitope contained within a peptide of amino acids 392-558 from the human protein shown in figure 3a.

35. A kit containing an antibody according to claim 33 or 34, further comprising at least one component that enables or supports the use of said antibody.

36. The use of an antibody according to claim 33 or 34 or a kit according to claim 35 for the detection of cells engaged in cell-cycle.

37. The use of an antibody according to claim 33 or 34 or a kit according to claim 35 for the diagnosis of malignant cells.


SEQUENCE LISTING

(1) GENERAL INFORMATION:

(i) APPLICANT:
(A) NAME: Introgene B.V.
(B) STREET: Lange Kleiweg 151
(C) CITY: Rijswijk
(D) STATE: Zuid-Holland
(E) COUNTRY: the Netherlands
(F) POSTAL CODE (ZIP): 2288 GJ

(ii) TITLE OF INVENTION: A novel transcription factor expressed in
cycling cells, nucleic acid molecules encoding said
transcription factor and its promoter region and uses
thereof.

(iii) NUMBER OF SEQUENCES: 28

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96203482.3

(2) INFORMATION FOR SEQ ID NO: 1:

(i) SEQUENCE CHARACTERISTICS:
(A) LENGTH: 6 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 1:

YAAACA 6

(2) INFORMATION FOR SEQ ID NO: 2:

(i) SEQUENCE CHARACTERISTICS:
(A) LENGTH: 6 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 2:

TAAACA 6

(2) INFORMATION FOR SEQ ID NO: 3:

(i) SEQUENCE CHARACTERISTICS:
(A) LENGTH: 20 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 3:

AARCCHCCHT AWTCNTAYAT 20

(2) INFORMATION FOR SEQ ID NO: 4:

(i) SEQUENCE CHARACTERISTICS:
(A) LENGTH: 21 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 4:

RTGYGTRATY GARTTCTGCC A 21

(2) INFORMATION FOR SEQ ID NO: 5:

(i) SEQUENCE CHARACTERISTICS:
(A) LENGTH: 64 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 5:

CTGGGTACCT CGAGTGAAGC TTGANNNNNN NNAANNNNNN NNGCGAATTC GGATCCGCGG 60

TAAC 64

(2) INFORMATION FOR SEQ ID NO: 6:

(i) SEQUENCE CHARACTERISTICS:
(A) LENGTH: 24 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 6:

CTGGGTACCT CGAGTGAAGC TTGA 24

(2) INFORMATION FOR SEQ ID NO: 7:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 7:

GTTACCGCGG ATCCGAATTC CC 22

(2) INFORMATION FOR SEQ ID NO: 8:

(i) SEQUENCE CHARACTERISTICS:
(A) LENGTH: 2640 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 8:

GAATTCCGCG TGGAGCAGAC GCGGCCTGTG AGGCTCAAAG CTTGC[?]ATTC [?]C[?]A[?]GGAGA 60
GCGAAAGCAC AGCCTCACGA TGAGAACCAG CCCCCGCCGG CCACTGATTC TCAAAAGACG 120
GAGGCTGCCC CTTCCTGTCC AGAATGCCCC GAGTGAAACA TCAGAGGAAG AAGCAAAGAG 180
GTCCCCTGCC CAGCCGGAGC CTGCTCCAGC ACAGGCCTCC CAAGAGGTGG CAGAGTCCAG 240
CTCTTGCAAA TTTCCAGCCG GAATCAAGAT TATCAACCAC CCCACCACGC CCAACACACA 300
AGTGGTGGCC ATCCCCAGCA ACGCTGATAT CCAGAGCATC ATCACAGCGC TAACTGCCAA 360
AGGGAAGGAG AGCGGCACGA GCGGGCCCAA CCGGTTCATC CTCATCAGCT CTGGGGGGCC 4[?]0
CTCCTCTCAC CCTTCCCAGC CTCAAGCCCA CAGCAGCCGG GATTCCAAGA GAGCAGAGGT 4[?]0
GATCACGGAG ACGTTGGGAC CGAAGCCAGC AGCTAAGGGT GTGCCTGTTC CCAAGCCTCC 540
TGGAGCCCCT CCAAGGCAAA GACAGGAGAG CTATGCTGGT GGTGAGGCGG CAGGCTGCAC 600
GCTGGACAAC AGCTTAACCA ATATCCAGTG GCTTGGAAAG ATGAGTTCTG ACGGGCTGGG 660
CCCCTGCAGC GTTAAGCAGG AACTGGAAGA GAAGGAGAAT TGTCACCTGG AGCAGAATCG 720
GGTTAAGGTT GAGGAGCCCT CAGGAGTGTC AACATCTTGG CAGGACTCTG TGTCTGAGAG 780
GCCACCCTAC TCTTATATGG CCATGATACA GTTTGCCATC AACAGCACTG AGAGAAAGCG 840
CATGACCTTG AAGGACATCT ACACTTGGAT TGAGGACCAC TTCCCTTACT TTAAGCACAT 900
TGCCAAGCCA GGCTGGAAGA ACTCTATTCG TCACAACCTT TCTCTCCATG ACATGTTTGT 960
TCGAGAGACA TCTGCCAATG GCAAGGTCTC CTTCTGGACC ATTCACCCAA GTGCCAATCG 1020
CCACTTGACA TTGGACCAAG TGTTTAAGCC ACTGGAACCA GGGTCTCCAC AATCGCCCGA 1080
GCACTTGGAA TCACAGCAGA AACGACCCAA TCCTGAGCTC CATAGAAATG TGACCATCAA 1140
AACTGAAATC CCACTGGGCG CACGGCGAAA GATGAAGCCT CTGCTCCCTC GGGTTAGCTC 1200
ATACCTGGAG CCCATCCAGT TCCCAGTGAA CCAGTCCCTG GTGTTACAGC CCTCAGTGAA 1260
GGTTCCCTTC CGTCTGGCAG CATCGCTTAT GAGCTCAGAG CTCGCCCGTC ATAGCAAGCG 1320
AGTCCGCATT GCACCCAAGG TGTTGCTATC CAGTGAAGGA ATAGCCCCGC TTCCTGCCAC 1380
AGAACCACCG AAGGAGGAGA AACCCCTGCT TGGAGGGGAA GGGCTGTTGC CTTTACTTCC 1440
TATTCAGTCC ATTAAGGAAG AAGAAATGCA GCCTGAGGAG GACATAGCAC ACTTAGAGAG 1500
GCCTATCAAA GTGGAGAGCC CTCCCTTGGA AGAGTGGCCC TCTCCGTGTG CATCGCTCAA 1560
AGAGGAGCTA TCCAACTCCT GGGAAGATTC TTCCTGCTCT CCTACCCCAA AGCCCAAGAA 1620
GTCCTACTGT GGGCTCAAGT CCCCAACGCG CTGTGTCTCA GAAATGCTGG TGACTAAGCG 1680
AAGAGAGAAA AGGGAGGTGA GCCGATCCCG GAGGAAACAG CACCTTCAGC CCCCCTGTCT 1740
GGATGAGCCT GACCTGTTCT TCCCAGAGGA CTCCAGCACA TTTCGGCCAG CCGTGGAGCT 1800

CCTGGCGGAG TCTTCCGAGC CTGCACCCCA CCTCACCTG[?] CCTCA[?]G[?]AG AG[?]GAGGACC 1860

TTTCAAGACC CCCATCAAGG AGACATTGCC TGTCTCCTCC ACTCCTAGCA AGTCTGTGCT 1920

CTCTAGAGAC CCTGAGTCCT GGAGGCTCAC ACCTCCAGCC AAAGTTGGGG GGTTAGATTT 1980

CAGCCCAGTA CGAACCCCCC AGGGTGCCTT TGGCCTTCTG CCTGACTCAC TGGGGTTGAT 2040

GGAGCTGAAT ACCACCCCTT TGAAAAGTGG TCCTCTCTTT GACTCGCCCC GGGAGCTCCT 2100

CAACTCAGAG CCCTTTGACC TGGCCTCTGA CCCCTTCGGC AGCCCTCCAC CACCACATGT 2160

GGAAGGCCCC AAGCCTGGCT CCCCCGAGCT GCAGATTCCC AGCCTTTCAG CCAACCGTTC 2220

TCTCACAGAA GGCCTTGTCC TGGACACAAT GAATGATAGC CTCAGCAAGA TCCTTCTAGA 2280

CATCAGTTTC CCTGGCCTGG AGGAGGACCC TCTGGGCCCT GACAACATCA ACTGGTCTCA 2340

GTTCATCCCT TAGCTGCGAT AGAGGCAAGG CCTTGCCCCT GCCACTCAAG CCGCCTGCTA 2400

TCCTGGCACT TGTGTGGCTC AGGGTACCCC AAGCCGTCTG AGGGAAGCTA GCAGGCAAGG 2460

GCTGAGCTGT GCCCTTTGAC CTAATTATGT CAAGATGATA GCCGCTACTA AGCCACACCA 2520

GGACCTATGC AAGCAGTAGG ATCCCCCAGA GTCCGAGTCC TCCACTCCCT GCTGGCAAGT 2580

GAAGTGGGTG TGACGAGCCA TGAGGACCAG GAAGTGCCCA TTAGTCACTC GGTGCTCCTG 2640

(2) INFORMATION FOR SEQ ID NO: 9:

(i) SEQUENCE CHARACTERISTICS:
(A) LENGTH: 757 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 9:

Met Arg Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Ala
            20                  25                  30

Lys Arg Ser Pro Ala Gln Pro Glu Pro Ala Pro Ala Gln Ala Ser Gln
        35                  40                  45

Glu Val Ala Glu Ser Ser Ser Cys Lys Phe Pro Ala Gly Ile Lys Ile
    50                  55                  60

Ile Asn His Pro Thr Thr Pro Asn Thr Gln Val Val Ala Ile Pro Ser

65                  70                  75                  80

Asn Ala Asp Ile Gln Ser Ile Ile Thr Ala Leu Thr Ala Lys Gly Lys
                85                  90                  95

Glu Ser Gly Thr Ser Gly Pro Asn Arg Phe Ile Leu Ile Ser Ser Gly
            100                 105                 110

Gly Pro Ser Ser His Pro Ser Gln Pro Gln Ala His Ser Ser Arg Asp
        115                 120                 125

Ser Lys Arg Ala Glu Val Ile Thr Glu Thr Leu Gly Pro Lys Pro Ala
    130                 135                 140

Ala Lys Gly Val Pro Val Pro Lys Pro Pro Gly Ala Pro Pro Arg Gln
145                 150                 155                 160

Arg Gln Glu Ser Tyr Ala Gly Gly Glu Ala Ala Gly Cys Thr Leu Asp
                165                 170                 175

Asn Ser Leu Thr Asn Ile Gln Trp Leu Gly Lys Met Ser Ser Asp Gly
            180                 185                 190

Leu Gly Pro Cys Ser Val Lys Gln Glu Leu Glu Glu Lys Glu Asn Cys
        195                 200                 205

His Leu Glu Gln Asn Arg Val Lys Val Glu Glu Pro Ser Gly Val Ser
    210                 215                 220

Thr Ser Trp Gln Asp Ser Val Ser Glu Arg Pro Pro Tyr Ser Tyr Met
225                 230                 235                 240

Ala Met Ile Gln Phe Ala Ile Asn Ser Thr Glu Arg Lys Arg Met Thr
                245                 250                 255

Leu Lys Asp Ile Tyr Thr Trp Ile Glu Asp His Phe Pro Tyr Phe Lys
            260                 265                 270

His Ile Ala Lys Pro Gly Trp Lys Asn Ser Ile Arg His Asn Leu Ser
        275                 280                 285

Leu His Asp Met Phe Val Arg Glu Thr Ser Ala Asn Gly Lys Val Ser
    290                 295                 300

Phe Trp Thr Ile His Pro Ser Ala Asn Arg His Leu Thr Leu Asp Gln
305                 310                 315                 320

Val Phe Lys Pro Leu Glu Pro Gly Ser Pro Gln Ser Pro Glu His Leu
                325                 330                 335

Glu Ser Gln Gln Lys Arg Pro Asn Pro Glu Leu His Arg Asn Val Thr
            340                 345                 350

Ile Lys Thr Glu Ile Pro Leu Gly Ala Arg Arg Lys Met Lys Pro Leu
        355                 360                 365

Leu Pro Arg Val Ser Ser Tyr Leu Glu Pro Ile Gln Phe Pro Val Asn
    370                 375                 380

Gln Ser Leu Val Leu Gln Pro Ser Val Lys Val Pro Phe Arg Leu Ala

385                 390                 395                 400

Ala Ser Leu Met Ser Ser Glu Leu Ala Arg His Ser Lys Arg Val Arg
                405                 410                 415

Ile Ala Pro Lys Val Leu Leu Ser Ser Glu Gly Ile Ala Pro Leu Pro
            420                 425                 430

Ala Thr Glu Pro Pro Lys Glu Glu Lys Pro Leu Leu Gly Gly Glu Gly
        435                 440                 445

Leu Leu Pro Leu Leu Pro Ile Gln Ser Ile Lys Glu Glu Glu Met Gln
    450                 455                 460

Pro Glu Glu Asp Ile Ala His Leu Glu Arg Pro Ile Lys Val Glu Ser
465                 470                 475                 480

Pro Pro Leu Glu Glu Trp Pro Ser Pro Cys Ala Ser Leu Lys Glu Glu
                485                 490                 495

Leu Ser Asn Ser Trp Glu Asp Ser Ser Cys Ser Pro Thr Pro Lys Pro
            500                 505                 510

Lys Lys Ser Tyr Cys Gly Leu Lys Ser Pro Thr Arg Cys Val Ser Glu
        515                 520                 525

Met Leu Val Thr Lys Arg Arg Glu Lys Arg Glu Val Ser Arg Ser Arg
    530                 535                 540

Arg Lys Gln His Leu Gln Pro Pro Cys Leu Asp Glu Pro Asp Leu Phe
545                 550                 555                 560

Phe Pro Glu Asp Ser Ser Thr Phe Arg Pro Ala Val Glu Leu Leu Ala
                565                 570                 575

Glu Ser Ser Glu Pro Ala Pro His Leu Ser Cys Pro Gln Glu Glu Gly
            580                 585                 590

Gly Pro Phe Lys Thr Pro Ile Lys Glu Thr Leu Pro Val Ser Ser Thr
        595                 600                 605

Pro Ser Lys Ser Val Leu Ser Arg Asp Pro Glu Ser Trp Arg Leu Thr
    610                 615                 620

Pro Pro Ala Lys Val Gly Gly Leu Asp Phe Ser Pro Val Arg Thr Pro
625                 630                 635                 640

Gln Gly Ala Phe Gly Leu Leu Pro Asp Ser Leu Gly Leu Met Glu Leu
                645                 650                 655

Asn Thr Thr Pro Leu Lys Ser Gly Pro Leu Phe Asp Ser Pro Arg Glu
            660                 665                 670

Leu Leu Asn Ser Glu Pro Phe Asp Leu Ala Ser Asp Pro Phe Gly Ser
        675                 680                 685

Pro Pro Pro Pro His Val Glu Gly Pro Lys Pro Gly Ser Pro Glu Leu
    690                 695                 700

Gln Ile Pro Ser Leu Ser Ala Asn Arg Ser Leu Thr Glu Gly Leu Val

705                 710                 715                 720

Leu Asp Thr Met Asn Asp Ser Leu Ser Lys Ile Leu Leu Asp Ile Ser
                725                 730                 735

Phe Pro Gly Leu Glu Glu Asp Pro Leu Gly Pro Asp Asn Ile Asn Trp
            740                 745                 750

Ser Gln Phe Ile Pro
        755

(2) INFORMATION FOR SEQ ID NO: 10:

(i) SEQUENCE CHARACTERISTICS:
(A) LENGTH: 3431 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 10:

CTGAAAGCTC CGGTGCCAGA CCCCACCCCC GGCCCCGGCC CGGACCCCCT CCCCTCCCGG      60

GATCCCCGGG TTCCCACCCC GCCCGCACCG CCGGGGACCC GGCCGGTCCG GCGCGAGCCC     120

CGTCCGGGCC CTGGCTCGGC CCCAGGTTGG AGGAGCCGGA GCCGCCTTCG GAGCTACGGC     180

CTAACGGCAG GCGGCGACTG CAGTCTGGAG GGTCCACACT TGTGATTCTC AATGGAGAGT     240

GAAAACGCAG ATTCATAATG AAAACTAGCC CCCGTCGGCC ACTGATTCTC AAAAGACGGA     300

GGCTGCCCCT TCCTGTTCAA AATGCCCCAA GTGAAACATC AGAGGAGGAA CCTAAGAGAT     360

CCCCTGCCCA ACAGGAGTCT AATCAAGCAG AGGCCTCCAA GGAAGTGGCA GAGTCCAACT     420

CTTGCAAGTT TCCAGCTGGG ATCAAGATTA TTAACCACCC CACCATGCCC AACACGCAAG     480

TAGTGGCCAT CCCCAACAAT GCTAATATTC ACAGCATCAT CACAGCACTG ACTGCCAAGG     540

GAAAAGAGAG TGGCAGTAGT GGGCCCAACA AATTCATCCT CATCAGCTGT GGGGGAGCCC     600

CAACTCAGCC TCCAGGACTC CGGCCTCAAA CCCAAACCAG CTATGATGCC AAAAGGACAG     660

AAGTGACCCT GGAGACCTTG GGACCAAAAC CTGCAGCTAG GGATGTGAAT CTTCCTAGAC     720

CACCTGGAGC CCTTTGCGAG CAGAAACGGG AGACCTGTGA TGGTGAGGCA GCAGGCTGCA     780

CTATCAACAA TAGCCTATCC AACATCCAGT GGCTTCGAAA GATGAGTTCT GATGGACTGG     840

GCTCCCGCAG CATCAAGCAA GAGATGGAGG AAAAGGAGAA TTGTCACCTG GAGCAGCGAC     900

AGGTTAAGGT TGAGGAGCCT TCGAGACCAT CAGCGTCCTG GCAGAACTCT GTGTCTGAGC     960

GGCCACCCTA CTCTTACATG GCCATGATAC AATTCGCCAT CAACAGCACT GACAGGAAGC 1020
GCATGACTTT GAAAGACATC TATACGTGGA TTGAGGACCA CTTTCCCTAC TTTAAGCACA 1080
TTGCCAAGCC AGGCTGGAAG AACTCCATCC GCCACAACCT TTCCCTGCAC GACATGTTTG 1140
TCCGGGAGAC GTCTGCCAAT GGCAAGGTCT CCTTCTGGAC CATTCACCCC AGTGCCAACC 1200
GCTACTTGAC ATTGGACCAG GTGTTTAAGC AGCAGAAACG ACCGAATCCA GAGCTCCGCC 1260
GGAACATGAC CATCAAAACC GAACTCCCCC TGGGCGCACG GCGGAAGATG AAGCCACTGC 1320
TACCACGGGT CAGCTCATAC CTGGTGCCTA TCCAGTTCCC GGTGAACCAG TCACTGGTGT 1380
TGCAGCCCTC GGTGAAGGTG CCATTGCCCC TGGCGGCTTC CCTCATGAGC TCAGAGCTTG 1440
CCCGCCATAG CAAGCGAGTG CGCATTGCCC CCAAGGTGCT GCTAGCTGAG GAGGGGATAG 1500
CTCCTCTTTC TTCTGCAGGA CCAGGGAAAG AGGAGAAACT CCTGTTTGGA GAAGGGTTTT 1560
CTCCTTTGCT TCCAGTTCAG ACTATCAAGG AGGAAGAAAT CCAGCCTGGG GAGGAAATGC 1620
CACACTTAGC GAGACCCATC AAAGTGGAGA GCCCTCCCTT GGAAGAGTGG CCCTCCCCGG 1680
CCCCATCTTT CAAAGAGGAA TCATCTCACT CCTGGGAGGA TTCGTCCCAA TCTCCCACCC 1740
CAAGACCCAA GAAGTCCTAC AGTGGGCTTA GGTCCCCAAC CCGGTGTGTC TCGGAAATGC 1800
TTGTGATTCA ACACAGGGAG AGGAGGGAGA GGAGCCGGTC TCGGAGGAAA CAGCATCTAC 1860
TGCCTCCCTG TGTGGATGAG CCGGAGCTGC TCTTCTCAGA GGGGCCCAGT ACTTCCCGCT 1920
GGGCCGCAGA GCTCCCGTTC CCAGCAGACT CCTCTGACCC TGCCTCCCAG CTCAGCTACT 1980
CCCAGGAAGT GGGAGGACCT TTTAAGACAC CCATTAAGGA AACGCTGCCC ATCTCCTCCA 2040
CCCCGAGCAA ATCTGTCCTC CCCAGAACCC CTGAATCCTG GAGGCTCACG CCCCCAGCCA 2100
AAGTAGGGGG ACTGGATTTC AGCCCAGTAC AAACCTCCCA GGGTGCCTCT GACCCCTTGC 2160
CTGACCCCCT GGGGCTGATG GATCTCAGCA CCACTCCCTT GCAAAGTGCT CCCCCCCTTG 2220
AATCACCGCA AAGGCTCCTC AGTTCAGAAC CCTTAGACCT CATCTCCGTC CCCTTTGGCA 2280
ACTCTTCTCC CTCAGATATA GACGTCCCCA AGCCAGGCTC CCCGGAGCCA CAGGTTTCTG 2340
GCCTTGCAGC CAATCGTTCT CTGACAGAAG GCCTGGTCCT GGACACAATG AATGACAGCC 2400
TCAGCAAGAT CCTGCTGGAC ATCAGCTTTC CTGGCCTGGA CGAGGACCCA CTGGGCCCTG 2460
ACAACATCAA CTGGTCCCAG TTTATTCCTG AGCTACAGTA GAGCCCTGCC CTTGCCCCTG 2520
TGCTCAAGCT GTCCACCATC CCGGGCACTC CAAGGCTCAG TGCACCCCAA GCCTCTGAGT 2580
GAGGACAGCA GGCAGGGACT GTTCTGCTCC TCATAGCTCC CTGCTGCCTG ATTATGCAAA 2640
AGTAGCAGTC ACACCCTAGC CACTGCTGGG ACCTTGTGTT CCCCAAGAGT ATCTGATTCC 2700
TCTGCTGTCC CTGCCAGGAG CTGAAGGGTG GGAACAACAA AGGCAATGGT GAAAAGAGAT 2760

TAGGAACCCC CCAGCCTGTT TCCATTCTCT GCCCACCAGT CTCTTACCTT CCCTGATCTT 2820

TGCAGGGTGG TCCGTGTAAA TAGTATAAAT TCTCCAAATT ATCCTCTAAT TATAAATGTA 2880

AGCTTATTTC CTTAGATCAT TATCCAGAGA CTGCCAGAAG GTGGGTAGGA TGACCTGGGG 2940

TTTCAATTGA CTTCTGTTCC TTGCTTTTAG TTTTGATAGA AGGGAAGACC TGCAGTGCAC 3000

GGTTTCTTCC AGGCTGAGGT ACCTGGATCT TGGGTTCTTC ACTGCAGGGA CCCAGACAAG 3060

TGGATCTGCT TGCCAGAGTC CTTTTTGCCC CTCCCTGCCA CCTCCCCGTG TTTCCAAGTC 3120

AGCTTTCCTG CAAGAAGAAA TCCTGGTTAA AAAAGTCTTT TGTATTGGGT CAGGAGTTGA 3180

ATTTGGGGTG GGAGGATGGA TGCAACTGAA GCAGAGTGTG GGTGCCCAGA TGTGCGCTAT 3240

TAGATGTTTC TCTGATAATG TCCCCAATCA TACCAGGGAG ACTGGCATTG ACGAGAACTC 3300

AGGTGGAGGC TTGAGAAGGC CGAAAGGGCC CCTGACCTGC CTGGCTTCCT TAGCTTGCCC 3360

CTCAGCTTTG CAAAGAGCCA CCCTAGGCCC CAGCTGACCG CATGGGTGTG AGCCAGCTTG 3420

AGAACACTAA C 3431

(2) INFORMATION FOR SEQ ID NO: 11:

(i) SEQUENCE CHARACTERISTICS:
(A) LENGTH: 747 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 11:

Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Pro
            20                  25                  30

Lys Arg Ser Pro Ala Gln Gln Glu Ser Asn Gln Ala Glu Ala Ser Lys
        35                  40                  45

Glu Val Ala Glu Ser Asn Ser Cys Lys Phe Pro Ala Gly Ile Lys Ile
    50                  55                  60

Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95

Glu Ser Gly Ser Ser Gly Pro Asn Lys Phe Ile Leu Ile Ser Cys Gly

            100                 105                 110

Gly Ala Pro Thr Gln Pro Pro Gly Leu Arg Pro Gln Thr Gln Thr Ser
        115                 120                 125

Tyr Asp Ala Lys Arg Thr Glu Val Thr Leu Glu Thr Leu Gly Pro Lys
    130                 135                 140

Pro Ala Ala Arg Asp Val Asn Leu Pro Arg Pro Pro Gly Ala Leu Cys
145                 150                 155                 160

Glu Gln Lys Arg Glu Thr Cys Asp Gly Glu Ala Ala Gly Cys Thr Ile
                165                 170                 175

Asn Asn Ser Leu Ser Asn Ile Gln Trp Leu Arg Lys Met Ser Ser Asp
            180                 185                 190

Gly Leu Gly Ser Arg Ser Ile Lys Gln Glu Met Glu Glu Lys Glu Asn
        195                 200                 205

Cys His Leu Glu Gln Arg Gln Val Lys Val Glu Glu Pro Ser Arg Pro
    210                 215                 220

Ser Ala Ser Trp Gln Asn Ser Val Ser Glu Arg Pro Pro Tyr Ser Tyr
225                 230                 235                 240

Met Ala Met Ile Gln Phe Ala Ile Asn Ser Thr Glu Arg Lys Arg Met
                245                 250                 255

Thr Leu Lys Asp Ile Tyr Thr Trp Ile Glu Asp His Phe Pro Tyr Phe
            260                 265                 270

Lys His Ile Ala Lys Pro Gly Trp Lys Asn Ser Ile Arg His Asn Leu
        275                 280                 285

Ser Leu His Asp Met Phe Val Arg Glu Thr Ser Ala Asn Gly Lys Val
    290                 295                 300

Ser Phe Trp Thr Ile His Pro Ser Ala Asn Arg Tyr Leu Thr Leu Asp
305                 310                 315                 320

Gln Val Phe Lys Gln Gln Lys Arg Pro Asn Pro Glu Leu Arg Arg Asn
                325                 330                 335

Met Thr Ile Lys Thr Glu Leu Pro Leu Gly Ala Arg Arg Lys Met Lys
            340                 345                 350

Pro Leu Leu Pro Arg Val Ser Ser Tyr Leu Val Pro Ile Gln Phe Pro
        355                 360                 365

Val Asn Gln Ser Leu Val Leu Gln Pro Ser Val Lys Val Pro Leu Pro
    370                 375                 380

Leu Ala Ala Ser Leu Met Ser Ser Glu Leu Ala Arg His Ser Lys Arg
385                 390                 395                 400

Val Arg Ile Ala Pro Lys Val Leu Leu Ala Glu Glu Gly Ile Ala Pro
                405                 410                 415

Leu Ser Ser Ala Gly Pro Gly Lys Glu Glu Lys Leu Leu Phe Gly Glu

            420                 425                 430

Gly Phe Ser Pro Leu Leu Pro Val Gln Thr Ile Lys Glu Glu Glu Ile
        435                 440                 445

Gln Pro Gly Glu Glu Met Pro His Leu Ala Arg Pro Ile Lys Val Glu
    450                 455                 460

Ser Pro Pro Leu Glu Glu Trp Pro Ser Pro Ala Pro Ser Phe Lys Glu
465                 470                 475                 480

Glu Ser Ser His Ser Trp Glu Asp Ser Ser Gln Ser Pro Thr Pro Arg
                485                 490                 495

Pro Lys Lys Ser Tyr Ser Gly Leu Arg Ser Pro Thr Arg Cys Val Ser
            500                 505                 510

Glu Met Leu Val Ile Gln His Arg Glu Arg Arg Glu Arg Ser Arg Ser
        515                 520                 525

Arg Arg Lys Gln His Leu Leu Pro Pro Cys Val Asp Glu Pro Glu Leu
    530                 535                 540

Leu Phe Ser Glu Gly Pro Ser Thr Ser Arg Trp Ala Ala Glu Leu Pro
545                 550                 555                 560

Phe Pro Ala Asp Ser Ser Asp Pro Ala Ser Gln Leu Ser Tyr Ser Gln
                565                 570                 575

Glu Val Gly Gly Pro Phe Lys Thr Pro Ile Lys Glu Thr Leu Pro Ile
            580                 585                 590

Ser Ser Thr Pro Ser Lys Ser Val Leu Pro Arg Thr Pro Glu Ser Trp
        595                 600                 605

Arg Leu Thr Pro Pro Ala Lys Val Gly Gly Leu Asp Phe Ser Pro Val
    610                 615                 620

Gln Thr Ser Gln Gly Ala Ser Asp Pro Leu Pro Asp Pro Leu Gly Leu
625                 630                 635                 640

Met Asp Leu Ser Thr Thr Pro Leu Gln Ser Ala Pro Pro Leu Glu Ser
                645                 650                 655

Pro Gln Arg Leu Leu Ser Ser Glu Pro Leu Asp Leu Ile Ser Val Pro
            660                 665                 670

Phe Gly Asn Ser Ser Pro Ser Asp Ile Asp Val Pro Lys Pro Gly Ser
        675                 680                 685

Pro Glu Pro Gln Val Ser Gly Leu Ala Ala Asn Arg Ser Leu Thr Glu
    690                 695                 700

Gly Leu Val Leu Asp Thr Met Asn Asp Ser Leu Ser Lys Ile Leu Leu
705                 710                 715                 720

Asp Ile Ser Phe Pro Gly Leu Asp Glu Asp Pro Leu Gly Pro Asp Asn
                725                 730                 735

Ile Asn Trp Ser Gln Phe Ile Pro Glu Leu Gln

740                    745

(2) INFORMATION FOR SEQ ID NO: 12:

    (i) SEQUENCE CHARACTERISTICS:
        (A) LENGTH: 115 amino acids
        (B) TYPE: amino acid
        (C) STRANDEDNESS: unknown
        (D) TOPOLOGY: unknown

    (ii) MOLECULE TYPE: protein

    (iii) HYPOTHETICAL: NO

    (xi) SEQUENCE DESCRIPTION: SEQ ID NO: 12:

Asp Ser Val Glu Arg Pro Pro Tyr Ser Tyr Met Ala Met Ile Gln Phe
1               5                   10                  15

Ala Ile Asn Ser Thr Glu Arg Lys Arg Met Thr Leu Lys Asp Ile Tyr
            20                  25                  30

Thr Trp Ile Glu Asp His Phe Pro Tyr Phe Lys His Ile Ala Lys Pro
        35                  40                  45

Gly Trp Lys Asn Ser Ile Arg His Asn Leu Ser His Asp Met Phe Val
    50                  55                  60

Arg Glu Thr Ser Ala Asn Gly Lys Val Ser Phe Trp Thr Ile His Pro
65                  70                  75                  80

Ser Ala Asn Arg Leu Thr Leu Asp Gln Val Phe Lys Pro Leu Glu Pro
                85                  90                  95

Gly Ser Pro Gln Ser Pro Glu His Leu Glu Ser Gln Gln Lys Arg Pro
            100                 105                 110

Asn Pro Glu
        115

(2) INFORMATION FOR SEQ ID NO: 13:

    (i) SEQUENCE CHARACTERISTICS:
        (A) LENGTH: 104 amino acids
        (B) TYPE: amino acid
        (C) STRANDEDNESS: unknown
        (D) TOPOLOGY: unknown

    (ii) MOLECULE TYPE: protein

    (iii) HYPOTHETICAL: NO

    (xi) SEQUENCE DESCRIPTION: SEQ ID NO: 13:

Pro Leu Thr His Ala Lys Pro Pro Tyr Ser Tyr Ile Ser Leu Ile Thr
1               5                   10                  15

Met Ala Ile Gln Gln Ala Pro Gly Lys Met Leu Thr Ser Glu Ile Tyr
            20                  25                  30

Gln Trp Ile Met Asp Leu Phe Pro Tyr Tyr Arg Glu Asn Gln Gln Arg
        35                  40                  45

Trp Gln Asn Ser Ile Arg His Ser Leu Ser Phe Asn Asp Cys Phe Val
    50                  55                  60

Lys Val Ala Arg Ser Pro Asp Lys Pro Gly Lys Gly Ser Tyr Trp Ala
65                  70                  75                  80

Leu His Pro Ser Ser Gly Asn Met Phe Glu Asn Gly Cys Tyr Leu Arg
                85                  90                  95

Arg Gln Lys Arg Phe Lys Leu Glu
            100

(2) INFORMATION FOR SEQ ID NO: 14:

(i) SEQUENCE CHARACTERISTICS:
(A) LENGTH: 104 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 14:

Pro Lys Asp Met Val Lys Pro Pro Tyr Ser Tyr Ile Leu Ile Thr Trp
1               5                   10                  15

Ala Ile Gln Asn Ala Pro Asp Lys Lys Ile Thr Leu Asn Gly Ile Tyr
            20                  25                  30

Gln Phe Ile Met Asp Arg Phe Pro Phe Tyr Arg Asp Asn Lys Gln Gly
        35                  40                  45

Trp Gln Asn Ser Ile Arg His Asn Leu Ser Leu Asn Glu Cys Phe Val
    50                  55                  60

Lys Val Pro Arg Asp Asp Lys Lys Pro Gly Lys Gly Ser Tyr Trp Thr
65                  70                  75                  80

Leu Asp Pro Asp Ser Tyr Asn Met Phe Glu Asn Gly Ser Phe Leu Arg
                85                  90                  95

Arg Arg Arg Arg Phe Lys Lys Lys
            100

(2) INFORMATION FOR SEQ ID NO: 15:

(i) SEQUENCE CHARACTERISTICS:
(A) LENGTH: 106 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 15:

Pro Lys Asp Asp Ser Lys Pro Pro Tyr Ser Tyr Ala Gln Leu Ile Val
1               5                   10                  15

Gln Ala Ile Thr Met Ala Pro Asp Lys Gln Leu Thr Leu Asn Gly Ile
            20                  25                  30

Tyr Thr His Ile Thr Lys Asn Tyr Pro Tyr Tyr Arg Thr Ala Asp Lys
        35                  40                  45

Gly Trp Gln Asn Ser Ile Arg His Asn Leu Ser Leu Asn Arg Tyr Phe
    50                  55                  60

Ile Lys Val Pro Arg Ser Gln Glu Glu Pro Gly Lys Gly Ser Phe Trp
65                  70                  75                  80

Arg Ile Asp Pro Ala Ser Glu Ser Lys Leu Ile Glu Gln Ala Phe Arg
                85                  90                  95

Lys Arg Arg Pro Arg Gly Val Pro Cys Phe
            100                 105

(2) INFORMATION FOR SEQ ID NO: 16:

(i) SEQUENCE CHARACTERISTICS:
(A) LENGTH: 105 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 16:

Lys Ser Ala Thr Ser Lys Pro Pro Tyr Ser Phe Ser Leu Leu Ile Tyr
1               5                   10                  15

Met Ala Ile Glu His Ser Pro Asn Lys Cys Leu Pro Val Lys Glu Ile
            20                  25                  30

Tyr Ser Trp Ile Leu Asp His Phe Pro Tyr Phe Ala Thr Ala Pro Thr
        35                  40                  45

Gly Trp Lys Asn Ser Val Arg His Asn Leu Ser Leu Asn Lys Cys Phe
    50                  55                  60

Gln Lys Val Glu Arg Ser His Gly Lys Val Asn Gly Lys Gly Ser Leu
65                  70                  75                  80

Trp Cys Val Asp Pro Glu Tyr Lys Pro Asn Leu Ile Gln Ala Leu Lys
                85                  90                  95

Lys Gln Pro Phe Ser Ser Ala Ser Ala
            100                 105

(2) INFORMATION FOR SEQ ID NO: 17:

(i) SEQUENCE CHARACTERISTICS:
(A) LENGTH: 105 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 17:

Leu Arg Arg Pro Glu Lys Pro Pro Tyr Ser Tyr Ile Ala Leu Ile Val
1               5                   10                  15

Met Ala Ile Gln Ser Ser Pro Ser Lys Arg Leu Thr Leu Ser Glu Ile
            20                  25                  30

Tyr Gln Phe Leu Gln Ala Arg Phe Pro Phe Phe Arg Gly Ala Tyr Gln
        35                  40                  45

Gly Trp Lys Asn Ser Val Arg His Asn Leu Ser Leu Asn Glu Cys Phe
    50                  55                  60

Ile Lys Leu Pro Lys Gly Leu Gly Arg Pro Gly Lys Gly His Tyr Trp
65                  70                  75                  80

Thr Ile Asp Pro Ala Ser Glu Phe Met Phe Glu Glu Gly Ser Phe Arg
                85                  90                  95

Arg Arg Pro Arg Gly Phe Arg Arg Lys
            100                 105

(2) INFORMATION FOR SEQ ID NO: 18:

(i) SEQUENCE CHARACTERISTICS:
(A) LENGTH: 2491 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 18:

CATGAGTTTG AGACCAGCCT GGGCAACACA GTGAAACCCA AATTCTACAA AAAATACAAA 60

AAATCAGGTG GGCACGGCTG CGTGCACCTG TAGTCCCAGC TACTCAGGAG GCTAAGGTGG 120

GCAGATCACT TGAGCCCAGG AGGTGGAGGC TGCAGTGAGC CGAGATCGTG CCACTGAACT 180

CCAGCCTGAG TGACAGAGCC AGAACCTTGT TTCAAAAAAA GAAAAGAAAA AAAAATTCAA 240

CAAGTTTAAA ACTGAACTTG GCCGCGCACA GTGGCTCATG CCTGTAATGC CAACACTGTG 300

AAAGGTGAAG GTGGGAGGAT CACTTGAACC GAATAGTTCA AGATCAATCT GGGCAACAGA 360

GTGAGACCCT GTCTCTACAA AAGATACAAA AATAAGATAG GTGTAGTGGC ATGTGCCTGT 420

AGGGGGTCCC AGCCATTCAG GAGGCTGAGG TGGGAGGATC ACCTGAGCCC AGCAGGTTGA 480

GGTTGTAGTG AGCGAAGGTC ATGCCACTGG ACGTCAGCCT GGGGAACAGA GCAAGACCCT 540

GTCTCTAAAA AATAAAAAGA TAAAGAAAAA TAAATATATA ATTAAAAAAA AAACTCATTC 600

TGAGCATTGC TTTGTAGTAA AAAAACAAAA ACTGAATTCA CGATCATTCC CCTGACAACC 660

CCCCGCCCGT CCCTTGGCCA AAGTGATTAT CTTCCAGGGC CCTTGGTCAG GGAATAGTGT 720

CATTATTCCT TCAGTTATGC AGCTGAAAAT CTTGACACTT CTACCTCCCA TCCAACCCAC 780

CATCAAGCCC TGTCAACTTT ACCTCTTAAA CATGTTTTCT TAATGCCCCT TTTTTACTCT 840

CTCTGTCTCC TTCTTTCTCT TCCTCTCTCT CTCTCCCTGC CACCCTTTTT CAAGCTCCAG 900

CAGATCTTGC CTGGAGTATT GCAACATCCA ACTGTTCTGC CCTAATCCAC TCTAGCAATC 960

CCCGCTATAG AGGGACCTTT TAAAATGCAA ATCTGACCTG GATTCACTTC TGCTTAAAGC 1020

TTATGACTGG TTTCCTTTAA TTCTTGGATA AGCACAAAAC TTCTCGGTAT GGCTAAGGCT 1080

CTGCTTCCTC CGGCTCCAGC TTCAACTCAC ACCACACTCC ATTCAGGTCG ATTAGCTCAA 1140

ACCCCTCAGA TATTTCTCAG CCTTTGTACT CACTGTGCTT CCTCCTGCCA TCCAGCCTTT 1200

GCACGCCTGA TTCCCTCTGC TTAGAGGAGT TCCCTCCTTT CTTTGTAAAA GGCATCAGAT 1260

CTCACACCAA GCTCTCATTA CAAAAGCCTT TCCTGTCCTA CCTCTCAAGA TCTCGTTATT 1320

GTAAATACTC AAAGTATCAT TAACCTCTAA TGTTTCAATT TACAGCAATA ATTCAACATT 1380

TGTTTGTTTT GGAGACGGTG TCTCCCTCTG TCGCCCAGGC TGGAGTGCAG TGGTGTGATC 1440

ATGGCTTACC ATAGCCTCCA CCTCTCGGCC TCAGGTGATC CTTCCACCTC AGCCTCCAGA 1500

GTAGCTGGGA CTACAGGCAC GCACCACCAC GCCCGGCTAA TTTTTAGTAG AGAAGCGGTT 1560

TCGCTATGTT GCCAGGCTGG TCTCGAACTC GTGACCTCAA CTGATCCACC CGCCTCGGCC 1620

TCCTAAAGTG CTGGGATTAC AGGCGTGAGC CACTATGCCC AGCCCACATT TGTTTATTTG 1680

ATTAAAATGT CTGTGCCCCT CTTCCAGGAT TGGGCTGTGA GCCCAGGGGA AGGAAAGAAC 1740

CTTGTCTGCC ATTGTATCTT CAGGGCCTAG CGGTGCCTGG CGCACAGCAG TTGCTCAACT 1800

AGACTGGTTG ACTAAGTCAA TAAATAAAGC ACTACGGTCT ATTATATCCG AAGGCTTGGC 1860

TTCGGGAGGG GCAAAAGACA GGTTTCGCGC TGAGGTAGGG TTCATGGTGC CGACATTTTT 1920

TTTCAAGATG GAAGAAAGCG GAGATAATAC GCAGCCCTCA AAGGAACTTA GTCTAATCGG 1980

GGGGAGCAGA CGATCGTTCA CTGTGGGAAA ATGGGGTACG ATTTCCCCCA GTGAGGAAAT 2040

CAACTAAAGC CGAGCTTTGA AAAGGGGAGC AGAGGAGCCT GAGGGGAAGC GGGGGCGTGT 2100

CGCCTGGCGT GACCAGCGCG GCAGGAAAAG CGGGCCCAGG GACCCGGGCC TGTCACGCCG 2160

CTTCCGCGCG TCCCCAAACT CTCCCTCGGC TCGCCCACCC ACGCGGCGGG GACCCCTCCG 2220

GCCCCTCCCC GGCCCCACGG CCACTTCTTC CCCCACAAGC CGGCCTGCGG TCCGCCTTAC 2280

CAGCCCGGGC CGGACGGGGC CGCAGCTCCT GGCAGACCGC ACAGCCTTCG AGCCCGGAAT 2340

GCCGAGACAA GGCCGGCGCC GATTGGCGAC GTTCCGTCAC GTGACCTTAA CGCTCCGCCG 2400

GCGCCAATTT CAAACAGCGG AACAAACTGA AAGCTCCGGT GCCAGACCCC ACCCCCGGCC 2460

CCGGCCCGGG ACCCCCTCCC TCCCGGGATC C 2491

(2) INFORMATION FOR SEQ ID NO: 19:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 19:

GANNNNNNNN AANNNNNNNN GG 22

(2) INFORMATION FOR SEQ ID NO: 20:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 20:

GATGTTTGTA AACATGTTAG GG 22

(2) INFORMATION FOR SEQ ID NO: 21:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 21:

GAAGTAAATA AACATGTTTG GG 22

(2) INFORMATION FOR SEQ ID NO: 22:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 22:

GATGGATCAT AACATGTTTA GG 22

(2) INFORMATION FOR SEQ ID NO: 23:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 23:

GATTGTTTAC AAATGCACTT GG 22

(2) INFORMATION FOR SEQ ID NO: 24:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 24:

GAATGTTTAT AAACATGTTT GG 22

(2) INFORMATION FOR SEQ ID NO: 25:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 25:

GATTGTTTAT AAACATGTTT GG 22

(2) INFORMATION FOR SEQ ID NO: 26:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 26:

GAATGTTAGT AAACTTGTTT GG 22

(2) INFORMATION FOR SEQ ID NO: 27:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 27:

GATTGTTTAC AAACATGTTT GG 22

(2) INFORMATION FOR SEQ ID NO: 28:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 28:

GAAATTTAGT AAACATGTTT GG 22